# EUROPEAN PATENT SPECIFICATION

(11) **EP 2 633 070 B1**
(45) Date of publication and mention of the grant of the patent: **13.08.2014**
(21) Application number: 11779582.3
(22) Date of filing: 28.10.2011
(51) Int. Cl.: C12Q 1/68

(54) **NEW MARKER OF BREAST TUMORS FROM THE LUMINAL-B SYBTYPE**
NEUE BRUSTKREBSMARKER FÜR DEN LUMINAL-B SUBTYP
NOUVEAU MARQUEUR DU CANCER DU SEIN DE SOUS TYPE LUMINAL B

(30) Priority: 29.10.2010 US 407947 P
(43) Date of publication of application: 04.09.2013
(73) Proprietor: Institut National de la Santé et de la Recherche Médicale, 75013 Paris (FR); Universite d'Aix Marseille, 13284 Marseille Cedex 07 (FR); Institut Paoli-Calmettes, 13009 Marseille (FR)
(72) Inventor: BIRNBAUM, Daniel, F-13273 Marseille (FR); CHAFFANET, Max, F-13273 Marseille Cedex 9 (FR); GINESTIER, Christophe, F-13273 Marseille Cedex 09A (FR); ADELAÏDE, José, F-13009 Marseille (FR); SIRCOULOMB, Fabrice, Toronto, ON M5G 1L7 (CA)
(74) Representative: Becker, Philippe
(86) International application number: PCT/EP2011/005450
(87) International publication number: WO 2012/055565

(56) References cited:
- ADELAIDE JOSE ET AL: "Integrated profiling of basal and luminal breast cancers", CANCER RESEARCH, vol. 67, no. 24, December 2007 (2007-12), pages 11565-11575, XP002668606, ISSN: 0008-5472
- KWEK S S ET AL: "Co-amplified genes at 8p12 and 11q13 in breast tumors cooperate with two major pathways in oncogenesis", ONCOGENE, vol. 28, no. 17, April 2009 (2009-04), pages 1892-1903, XP002668607, ISSN: 0950-9232
- WILLIAMS SARAH V ET AL: "High-Resolution Analysis of Genomic Alteration on Chromosome Arm 8p in Urothelial Carcinoma", GENES CHROMOSOMES & CANCER, vol. 49, no. 7, July 2010 (2010-07), pages 642-659, XP002668608, ISSN: 1045-2257
- NAKAMURA MAKO ET AL: "Nlz1/Znf703 acts as a repressor of transcription", BMC DEVELOPMENTAL BIOLOGY, BIOMED CENTRAL LTD., LONDON, GB, vol. 8, no. 1, 12 November 2008 (2008-11-12), page 108, XP021041944, ISSN: 1471-213X, DOI: 10.1186/1471-213X-8-108
- MELCHOR LORENZO ET AL: "Genomic analysis of the 8p11-12 amplicon in familial breast cancer", INTERNATIONAL JOURNAL OF CANCER, vol. 120, no. 3, February 2007 (2007-02), pages 714-717, XP002668609, ISSN: 0020-7136
- POLE J C M ET AL: "High-resolution analysis of chromosome rearrangements on 8p in breast, colon and pancreatic cancer reveals a complex pattern of loss, gain and translocation", ONCOGENE, vol. 25, no. 41, September 2006 (2006-09), pages 5693-5706, XP002668610, ISSN: 0950-9232
- SIRCOULOMB FABRICE ET AL: "ZNF703 gene amplification at 8p12 specifies luminal B breast cancer", EMBO MOLECULAR MEDICINE, vol. 3, no. 3, March 2011 (2011-03), pages 153-166, XP002668611,
- HOLLAND DANIEL G ET AL: "ZNF703 is a common Luminal B breast cancer oncogene that differentially regulates luminal and basal progenitors in human mammary epithelium", EMBO MOLECULAR MEDICINE, vol. 3, no. 3, March 2011 (2011-03), pages 167-180, XP002668612,
- KRISTENSEN VESSELA N: "Divide and conquer: the genetic basis of molecular subclassification of breast cancer", EMBO MOLECULAR MEDICINE, vol. 3, no. 4, April 2011 (2011-04), pages 183-185, XP002668613,

### SEQUENCE LISTING

<110> Birnbaum, Daniel
<120> NEW MARKER OF BREAST TUMORS FROM THE LUMINAL-B SUBTYPE
<130> IPS-B-0010-PCT
<150> US 61/407,947
   <151> 2010/10/29
<160> 4
<170> PatentIn version 3.5
<210> 1
   <211> 2174
   <212> DNA
   <213> Homo sapiens
<400> 1
   gtttttgtgt tgctagccgg ggccagcggc ggtggcggcg gcggcggagg cgtcggtgga      60
   ggaggggagg cggcgaggag gcgcagctcc cgctgcaccg cgatcgacgc tgcggagcga     120
   gcccacccgc cccgggagct cgcctccccg gtgctccccc gccctccccg cccccccagc     180
   ggcgctgcct cctccaaatg agcgattcgc ccgctggatc taacccaagg acacccgaaa     240
   gcagcggcag cggcagcggc ggcggcggga agaggccggc ggtgccggca gcggtgtccc     300
   tcttgccacc ggcggacccc ctgcgccagg cgaaccggct cccgatcagg gtcctgaaga     360
   tgctgagcgc tcacaccggt cacctcctgc acccggagta cctgcagccg ctgtcctcca     420
   ctcccgtcag ccccattgag ctggacgcca agaagagccc cttggcgctg ctggctcaga     480
   cctgctcgca gatcggcaag ccggacccgc cgccctcctc caaactcaac tcggtggcgg     540
   cggcggccaa cgggctggga gcggagaagg accccggccg ctcagccccg ggcgccgcct     600
   ccgcagccgc ggccctgaag cagctggggg actcaccggc cgaggacaag tccagcttca     660
   agccctactc caagggctcc ggcggcggcg actcccgcaa agacagcggc tcctcctcgg     720
   tgtcttccac ctcctcctcg tcctcctcgt ccccgggaga caaggcgggc ttcagggtcc     780
   ccagcgccgc ctgcccgccc tttcccccgc atggagcgcc ggtctccgca tcctcgtcct     840
   cgtcgtcgcc cggcggctcc cgcggcggct ccccgcacca ctctgactgc aagaacggcg     900
   gcggggttgg cggcggggag ctggacaaga aagaccagga gcccaagccc agcccggagc     960
   cggcagccgt gagccgcggc ggcggtgggg agcccggggc gcacggtggc gccgagtccg    1020
   gggcctccgg gcgcaagtcc gagccgccct cggcgctggt gggggccggc cacgtggcgc    1080
   cggtgtctcc ctacaagccg ggccactcgg tgttcccgct gccgccctcc agcattggct    1140
   accacggctc catcgtgggc gcctacgccg gctacccgtc tcagttcgtg cctggcctgg    1200
   atcctagcaa gtccggcctc gtgggaggcc agctgtctgg gggcctgggc ctgccgccgg    1260
   gcaagccccc cagctccagc ccgctcaccg gggcctcccc gccctccttc ctgcagggat    1320
   tatgccgcga cccctattgc ttgggaggtt accacggcgc ctcgcacctc ggcggctcca    1380
   gctgctccac ctgcagcgcg cacgaccctg ccgggcccag cctgaaggcg gggggctacc    1440
   cgctggtgta ccccgggcac ccgctgcagc ccgccgcgct ctcgtccagc gccgcccagg 1500
   ccgcgctccc cggccacccg ctctacacct acggcttcat gctgcagaac gaaccgctgc 1560
   cgcacagctg caactgggtg gcagccagtg ggccgtgcga caagcgcttc gccacctcgg 1620
   aggagctgct cagccaccta cggacccaca cggccctgcc gggagccgag aaacttctgg 1680
   ccgcctaccc cggggcctcg ggcctgggca gcgccgccgc cgccgccgcc gccgccgcct 1740
   cctgccatct gcacctcccc ccgcccgccg cccccggcag ccccgggtcg ctgtccttgc 1800
   ggaatccaca cactttgggc ctaagccggt accaccccta tggcaagagc cacttatcca 1860
   cagcgggggg cctggccgtg ccgtccctcc ccacagccgg accctactat tcgccatacg 1920
   cgctgtatgg acagagacta gcttcagcct cggcgctggg ataccagtaa ctacagctct 1980
   tcctccaccc cagccccctc accctcctcc ctctccctcc tcctccctcc ccacctgccg 2040
   tcgccgctgc aacctccact actgcttgac cctgccggga ttccccaccc aacccttccc 2100
   caccggactg tgtatttatt tactataatg ttagcttaca agctgggaat ataagtgcat 2160
   taacggccca catg 2174
<210> 2
   <211> 590
   <212> PRT
   <213> Homo sapiens
<400> 2
   Met Ser Asp Ser Pro Ala Gly Ser Asn Pro Arg Thr Pro Glu Ser Ser
   1               5                   10                  15
   Gly Ser Gly Ser Gly Gly Gly Gly Lys Arg Pro Ala Val Pro Ala Ala
               20                  25                  30
   Val Ser Leu Leu Pro Pro Ala Asp Pro Leu Arg Gln Ala Asn Arg Leu
           35                  40                  45
   Pro Ile Arg Val Leu Lys Met Leu Ser Ala His Thr Gly His Leu Leu
       50                  55                  60
   His Pro Glu Tyr Leu Gln Pro Leu Ser Ser Thr Pro Val Ser Pro Ile
   65                  70                  75                  80
   Glu Leu Asp Ala Lys Lys Ser Pro Leu Ala Leu Leu Ala Gln Thr Cys
                   85                  90                  95
   Ser Gln Ile Gly Lys Pro Asp Pro Pro Pro Ser Ser Lys Leu Asn Ser
               100                 105                 110
   Val Ala Ala Ala Ala Asn Gly Leu Gly Ala Glu Lys Asp Pro Gly Arg
           115                 120                 125
   Ser Ala Pro Gly Ala Ala Ser Ala Ala Ala Ala Leu Lys Gln Leu Gly
       130                 135                 140
   Asp Ser Pro Ala Glu Asp Lys Ser Ser Phe Lys Pro Tyr Ser Lys Gly
   145 150 155 160
   Ser Gly Gly Gly Asp Ser Arg Lys Asp Ser Gly Ser Ser Ser Val Ser
   165 170 175
   Ser Thr Ser Ser Ser Ser Ser Ser Ser Pro Gly Asp Lys Ala Gly Phe
   180 185 190
   Arg Val Pro Ser Ala Ala Cys Pro Pro Phe Pro Pro His Gly Ala Pro
   195 200 205
   Val Ser Ala Ser Ser Ser Ser Ser Ser Pro Gly Gly Ser Arg Gly Gly
   210 215 220
   Ser Pro His His Ser Asp Cys Lys Asn Gly Gly Gly Val Gly Gly Gly
   225 230 235 240
   Glu Leu Asp Lys Lys Asp Gln Glu Pro Lys Pro Ser Pro Glu Pro Ala
   245 250 255
   Ala Val Ser Arg Gly Gly Gly Gly Glu Pro Gly Ala His Gly Gly Ala
   260 265 270
   Glu Ser Gly Ala Ser Gly Arg Lys Ser Glu Pro Pro Ser Ala Leu Val
   275 280 285
   Gly Ala Gly His Val Ala Pro Val Ser Pro Tyr Lys Pro Gly His Ser
   290 295 300
   Val Phe Pro Leu Pro Pro Ser Ser Ile Gly Tyr His Gly Ser Ile Val
   305 310 315 320
   Gly Ala Tyr Ala Gly Tyr Pro Ser Gln Phe Val Pro Gly Leu Asp Pro
   325 330 335
   Ser Lys Ser Gly Leu Val Gly Gly Gln Leu Ser Gly Gly Leu Gly Leu
   340 345 350
   Pro Pro Gly Lys Pro Pro Ser Ser Ser Pro Leu Thr Gly Ala Ser Pro
   355 360 365
   Pro Ser Phe Leu Gln Gly Leu Cys Arg Asp Pro Tyr Cys Leu Gly Gly
   370 375 380
   Tyr His Gly Ala Ser His Leu Gly Gly Ser Ser Cys Ser Thr Cys Ser
   385 390 395 400
   Ala His Asp Pro Ala Gly Pro Ser Leu Lys Ala Gly Gly Tyr Pro Leu
   405 410 415
   Val Tyr Pro Gly His Pro Leu Gln Pro Ala Ala Leu Ser Ser Ser Ala
   420 425 430
   Ala Gln Ala Ala Leu Pro Gly His Pro Leu Tyr Thr Tyr Gly Phe Met
   435 440 445
   Leu Gln Asn Glu Pro Leu Pro His Ser Cys Asn Trp Val Ala Ala Ser
   450 455 460
   Gly Pro Cys Asp Lys Arg Phe Ala Thr Ser Glu Glu Leu Leu Ser His
   465 470 475 480
   Leu Arg Thr His Thr Ala Leu Pro Gly Ala Glu Lys Leu Leu Ala Ala
   485 490 495
   Tyr Pro Gly Ala Ser Gly Leu Gly Ser Ala Ala Ala Ala Ala Ala Ala
   500 505 510
   Ala Ala Ser Cys His Leu His Leu Pro Pro Pro Ala Ala Pro Gly Ser
   515 520 525
   Pro Gly Ser Leu Ser Leu Arg Asn Pro His Thr Leu Gly Leu Ser Arg
   530 535 540
   Tyr His Pro Tyr Gly Lys Ser His Leu Ser Thr Ala Gly Gly Leu Ala
   545 550 555 560
   Val Pro Ser Leu Pro Thr Ala Gly Pro Tyr Tyr Ser Pro Tyr Ala Leu
   565 570 575
   Tyr Gly Gln Arg Leu Ala Ser Ala Ser Ala Leu Gly Tyr Gln
   580 585 590
<210> 3
   <211> 25
   <212> DNA
   <213> Artificial Sequence
<220>
   <223> PCR primer
<400> 3
   tcaccatgag cgattcgccc gctgg 25
<210> 4
   <211> 21
   <212> DNA
   <213> Artificial Sequence
<220>
   <223> PCR primer
<400> 4
   cctggtatcc cagcgccgag g 21

## Description

### Field of the Invention

The present invention relates to cancer diagnosis and more particularly, to method and kit useful for identifying patients suffering from breast cancer with poor prognosis.

### Background of the Invention

Despite improvements in the treatment of breast cancer, resistance to therapy is a major clinical problem. The molecular taxonomy of breast cancer based on transcriptional profiling has led to a better classification of tumors and pointed out new potential therapeutic targets.

Breast cancers are currently classified into five major molecular subtypes: luminal *A*, luminal B, basal, ERBB2-like, and normal-like *(*SORLIE et al., Proc. Natl. Acad. Sci. U.S.A., vol.100, p:8418-8423, 2003;SORLIE et al., Proc. Natl. Acad. Sci. U.S.A., vol.98, p: 10869-10874,2001). Molecular subtypes are associated with various clinical outcomes, and treatment strategies are being tailored to corresponding groups of patients (e.g. anti-ERBB2 therapies for patients with ERBB2-like tumors or hormone therapy for patients with luminal tumors).

Although luminal B tumors express hormone receptors (estrogen and progesterone), the risk of relapse is dramatically increased in women treated by hormone therapy for a luminal B tumor compared to women with a luminal A tumor. Little is known about specific signaling pathways deregulated in luminal B tumors. This molecular subtype is characterized by the expression of hormone receptors combined with high proliferative index. The specific gene expression signature associated with luminal B tumors is enriched in genes that drive the proliferation of cancer cells, such as *CCNE, MKI67,* or *MYBL2* (SORLIE *et al.,* abovementioned, 2001) and mitotic kinases (FINETTI et al., Cancer Res., 2008). Moreover, the functional loss of the retinoblastoma tumor suppressor gene (*RB1*) seems to be associated with the luminal B phenotype (HERSCHKOWITZ et al., Breast Cancer Res, vol.10, p:R75, 2008; JIANG et al., J. Clin. Invest., 2010).

The publication by Adelaide et al., Cancer Research, vol. 67, no. 24, pages 11565 - 11575, discloses on page 11568 the subject-matter that among 43 highly amplified genes associated with either basal or luminal subtype, 8 genes exhibited up-regulated expression in relation to amplification, and amongst said 8 genes is also mentioned the gene ZNF703. The text goes on stating that these 8 genes amongst which is ZNF703 were all associated with the luminal subtype. However, the luminal type is not further differentiated into e.g. luminal-A or luminal-B type. Therefore, this disclosure of does not anticipate the features of the underlying.

Finally, the identification of key mechanisms that regulate luminal B biology will thus lead to a better diagnostic and treatment of luminal tumors.

### Summary of the invention

The inventors have shown here that luminal B tumors display specific amplifications, including amplification of the 8p11-12 region in four subamplicons, A1 to A4.

The inventors have also shown that *ZNF703,* which is included in A1, is significantly amplified in luminal B tumors and that *ZNF703* mRNA and protein over-expression is associated with luminal B tumors and defines patients with poor clinical outcome.

Their results suggest that ZNF703 plays a role in the regulation of luminal B cancer stem cell population by controling key cell processes and has three important features that may explain its involvement in mammary oncogenesis.

First, *ZNF703* transcription is induced by estrogen stimulation. In agreement, *ZNF703* has been identified as an ER-regulated gene (LIN et al., PLoS Genet., vol.3, p:e87, 2007;LIN et a/., Genome Biol., vol.5, p:R66, 2004). In contrast, the other 8p12 potential oncogenes have not been shown to be regulated by ER. This could explain the association of A1 *(ZNF703)* amplification with the luminal B subtype. The 8p11-12 region is often co-amplified with the 11q13 region, which comprises the Cyclin D-encoding locus *CCND1. CCND1* is also under the control of ER. ZNF703 and CCND1 may thus function in the same pathway and potentiate each other. KWEK *et al.* have shown that CCND1 overexpression increases *ZNF703* expression (KWEK et a/., Oncogene, vol.28, p:1892-1903, 2009).

Second, the ZNF703 protein may be a cofactor in a nuclear corepressor complex composed of NCOR2, PHB2 and DCAF7. Corepressors control the action of many transcription factors including ER. Interestingly, ZNF703 overexpression represses the expression of luminal markers and ER-associated proteins such as GATA3 and FOXA1. Moreover, ZNF703-overexpressing cells display RB1 phosphorylation, P27 ^{kip1} downregulation, E2F1 activation and Cyclin E activation. ZNF703 interaction with DCAF7 may explain its role on E2F1 signaling. DCAF7 is a CUL4/DDB1 interacting protein that activates SKP2 to induce P27^{Kip1} proteolysis. Interestingly, the *DCAF7* gene is located in chromosomal region 17q23, which is often gained or amplified in luminal B breast cancers. Our GSEA showed that the same E2F1 activation/ER repression balance is prominent in luminal B tumors.

Third, *ZNF703* overexpression increases the formation of primary and secondary tumorspheres suggesting a role in the control of cancer stem cell self-renewal. Moreover, the gene expression program activated by ZNF703 overexpression is enriched in genes related to stem cell biology. It has been proposed that luminal tumors might arise through transformation of an ER-positive luminal progenitor. The oncogenic capacity of ZNF703 could thus be exerted on ER-positive mammary progenitors and contribute to re-activation of a self-renewal program.

All these specific features may explain the association of *ZNF703* amplification with the luminal B subtype and its frequent association with *CCND1.*

Thus, a first object of the invention is directed to an *in vitro* method for diagnosing a breast cancer from the luminal-B subtype in a female, which comprises the steps of analyzing a biological sample from said female by:
i) determining the copies number of the Zinc Finger Protein 703 gene (*ZNF703* gene), and/or
ii) determining the expression of the *ZNF703* gene,
wherein an increased copies number and/or an over-expression of the ZNF703 gene is indicative of a luminal B tumor.

A second object of the invention is directed to a kit for diagnosing a breast cancer from the luminal-B subtype in a female comprising at least one nucleic acid probe or oligonucleotide or at least one antibody, which can be used in a method as defined previously, for determining the copies number of the ZNF703 gene, and/or determining the expression of the ZNF703 gene.

Finally, a third object of the invention is directed to the use, for diagnosing a breast cancer from the luminal-B subtype in a female, of the abovementioned kit.

### Description of the drawings

The figure 1 shows that *ZNF703* is a target gene of the 8p12 amplification in luminal B breast tumors.
The figure 2 shows the identification and characterization of ZNF703 nuclear domain.
The figure 3 shows that *ZNF703* overexpression regulates cancer stem cell biology in MCF7 cell line.
The figure 4 shows that the ZNF703 overexpression regulates ER and E2F1 transcriptional activity.
The figure 5 shows that luminal B tumors present an activation of E2F1 transcriptional program and a decrease in the activity of ER-related transcription factors.

### Detailed description of the invention

In a first aspect of the invention, there is provided an *in vitro* method for diagnosing a breast cancer from the luminal-B subtype in a female, which comprises the steps of analyzing a biological sample from said female by:
i) determining the copies number of the Zinc Finger Protein 703 gene (*ZNF703* gene), and/or
ii) determining the expression of the *ZNF703* gene,
wherein an increased copies number and/or an over-expression of the *ZNF703* gene is indicative of a luminal B tumor.

Since luminal B subtype is associated with poor prognosis, the female diagnosed with breast cancer from the luminal-B subtype are preferably not treated with anti-estrogens, but with more aggressive treatments (chemotherapy).

Preferably, the method of the invention further comprises the step of determining the expression of the estrogen receptor, wherein the expression of the estrogen receptor is indicative of a luminal B tumor.

As used herein, the term "female" refers to a mammal, preferably a woman.

Said female may be a healthy, but the method of the invention is particularly useful for testing a female thought to develop or developing a breast cancer. In that case, the method of the invention enables to diagnose a breast cancer from the luminal-B subtype.

As used herein, the expression "biological sample" refers to a breast tissue sample, preferably a breast tumor sample.

Said biological sample can be obtained by various techniques, preferably by biopsy, more preferably by a minimally invasive technique or selected from core biopsy, excisional biopsy, ductal lavage simple, a fine needle aspiration sample or from cells micro dissected from the sample.

Little is known about the ZNF703 protein for "Zinc finger protein 703", also known as ZNF503L and FLJ14299, which is encoded by a gene located on the 8p12 chromosomal region, more specifically on the 8p11.23 chromosomal region.

The Zinc Finger Protein 703 gene (*ZNF703* gene), also known as FLJ14299, ZEPPO1, ZNF5O3L or ZPO1, is referenced with the accession number ID 80139, and its cDNA (Accession number NM_025069, SEQ ID n°1) is coding for a protein of 590 amino acids (Accession number NP_079345, SEQ ID n°2).

In a preferred embodiment of the invention, the method comprises the step of determining the copies number of the *ZNF703* gene.

This step of determining the copies number of the *ZNF703* gene can be done by well known methods such as Southern blot, quantitative PCR, DNA sequencing or fluorescence in situ hybridization (FISH). Primers or probes can be simply designed in view of the sequence of the known *ZNF703* gene.

As used herein, the expression "an increased copies number of the *ZNF703* gene" refers to a copies number of the *ZNF703* gene by genome, which is superior to the two alleles of the *ZNF703* gene.

In another preferred embodiment of the invention, the method comprises the step of determining the expression of the *ZNF703* gene.

Methods for analyzing the expression of a gene are well known for the man skilled in the art.

In a particular embodiment of the invention, the expression of the *ZNF703* gene is assessed by analyzing the expression of mRNA transcript or mRNA precursors, such as nascent RNA, of said gene.

Such analysis can be assessed by preparing mRNA/cDNA from cells in a biological sample from a subject, and hybridizing the mRNA/cDNA with a reference polynucleotide. The prepared mRNA/cDNA can be used in hybridization or amplification assays that include, but are not limited to, Northern analyses, polymerase chain reaction analyses, such as quantitative PCR (TAQMAN), and probes arrays.

Advantageously, the analysis of the expression level of mRNA transcribed from the *ZNF703* gene involves the process of nucleic acid amplification, e. g., by RT-PCR (the experimental embodiment set forth in U. S. Patent No. 4,683, 202), ligase chain reaction (BARANY, Proc. Natl. Acad. Sci. USA, vol.88, p: 189-193, 1991), self sustained sequence replication (GUATELLI et al., Proc. Natl. Acad. Sci. USA, vol.87, p: 1874-1878, 1990), transcriptional amplification system (KWOH et al., 1989, Proc. Natl. Acad. Sci. USA, vol.86, p: 1173-1177, 1989), Q-Beta Replicase (LIZARDI et al., Biol. Technology, vol.6, p: 1197, 1988), rolling circle replication (U. S. Patent No. 5,854, 033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3'regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

In another particular embodiment, the expression of the *ZNF703* gene is assessed by analyzing the expression of the ZNF703 protein translated from said gene.

Such analysis can be assessed using an antibody (*e.g.,* a radio-labeled, chromophore-labeled, fluorophore-labeled, or enzyme-labeled antibody), an antibody derivative (*e.g.,* an antibody conjugate with a substrate or with the protein or ligand of a protein of a protein/ligand pair (*e.g.,* biotin-streptavidin)), or an antibody fragment (*e.g.,* a single-chain antibody, an isolated antibody hypervariable domain, *etc.*) which binds specifically to the protein translated from the *ZNF703* gene. Said analysis can be assessed by a variety of techniques well known by one of skill in the art including, but not limited to, enzyme immunoassay (EIA), radioimmunoassay (RIA), Western blot analysis, immunohistochemistry and enzyme linked immunoabsorbant assay (ELISA).

Polyclonal antibodies can be prepared by immunizing a suitable animal, such as mouse, rabbit or goat, with the ZNF703 protein (SEQ ID n°2) or a fragment thereof (e.g., at least 10 or 15 amino acids). The antibody titer in the immunized animal can be monitored over time by standard techniques, such as with an ELISA using immobilized polypeptide. At an appropriate time after immunization, e.g, when the specific antibody titers are highest, antibody producing cells can be obtained from the animal and used to prepare monoclonal antibodies (mAb) by standard techniques, such as the hybridoma technique originally described by KOHLER and MILSTEIN (Nature, vol.256, p:495-497, 1975), the human B cell hybridoma technique (KOZBOR et al., Immunol., vol.4, p: 72, 1983), the EBV- hybridoma technique (COLE et al., In Monoclonal Antibodies and Cancer Therapy, Alan R. Liss,Inc., p: 77-96, 1985) or trioma techniques. The technology for producing hybridomas is well known (see generally Current Protocols in Immunology, COLIGAN et al. ed. , John Wiley & Sons, New York, 1994). Hybridoma cells producing the desired monoclonal antibody are detected by screening the hybridoma culture supernatants for antibodies that bind the polypeptide of interest, e.g., using a standard ELISA.

Commercial ZNF703 antibodies are available, as an example, one can cite the ones available from ABCAM, ABNOVA CORPORATION, ATLAS ANTIBODIES, AVIVA SYSTEMS BIOLOGY, EVEREST BIOTECH, GENETEX, GENWAY BIOTECH, INC., IMGENEX, LIFESPAN BIOSCIENCES, NOVUS BIOLOGICALS, PROSCI, INC, SANTA CRUZ BIOTECHNOLOGY, INC., or SIGMA-ALDRICH.

Alternatively, the step of determining the expression of the *ZNF703* gene can done indirectly by determining the expression of at least 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185 or 189 of the 189 genes listed in table 1, wherein the induction and/or downregulation of said genes are indicative of the expression of the *ZNF703* gene.

In fact, the examples established that a gene signature can be associated with *ZNF703* gene expression.

As used herein, the expression "over-expression of the *ZNF703* gene" occurs when the transcription and/or the translation of the gene leads to an expression level in a biological sample that is at least 20% superior to the normal level of expression of said gene, preferably at least 50% superior to the normal level of expression of said gene, and most preferably at least 100% superior to the normal level of expression of said gene.

Therefore, the method of the invention may comprise comparing the level of expression of the *ZNF703* gene in a biological sample from a female with its expression level in a control (i.e., normal expression level). A significantly greater level of expression of said gene in the biological sample of a female as compared to the normal expression level is an indication that the female suffers from a breast cancer of luminal-B subtype.

As used herein, a "control" corresponds preferably to a control sample comprising non-tumoral cells. Preferably, said control corresponds to normal breast tissues.

Thus, the "normal" level of expression of the *ZNF703* gene is the level of expression of said gene in a biological sample of non-tumoral cell. Preferably, said normal level of expression is assessed in a control sample and preferably, the average expression level of said gene in several control samples.

Determining the normal expression of the *ZNF703* gene may be assessed by any of a wide variety of well-known methods for detecting expression of a transcribed nucleic acid or translated protein as previously described.

In a second aspect, the present invention refers to a kit for diagnosing a breast cancer with the luminal-B subtype in a female comprising at least one nucleic acid probe or oligonucleotide or at least one antibody, which can be used in a method as defined previously, for determining the copies number of the *ZNF703* gene, and/or determining the expression of the ZNF703 gene or protein.

Preferably, the oligonucleotide is at least one PCR primer, preferably a set of PCR primers is provided, which allows amplifying the *ZNF703* gene or a fragment thereof. The skilled person readily provides such an oligonucleotide or set of PCR primers which allows to amplify a region of the *ZNF703* gene, provided that the nucleic acid sequence of *ZNF703* is well known (Current Protocols in Molecular Biology; edited by Fred M. Ausubel et al., supra).

As used herein, the term "kit" refers to any delivery system for delivering materials. In the context of reaction assays, such delivery systems include systems that allow for the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. As used herein, the term "fragmented kit" refers to delivery systems comprising two or more separate containers that each contains a subportion of the total kit components. The containers may be delivered to the intended recipient together or separately. For example, a first container may contain an enzyme for use in an assay, while a second container contains oligonucleotides. The term "fragmented kit" is intended to encompass kits containing Analyte specific reagents (ASR's) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

The present kits can also include one or more reagents, buffers, hybridization media, nucleic acids, primers, nucleotides, probes, molecular weight markers, enzymes, solid supports, databases, computer programs for calculating dispensation orders and/or disposable lab equipment, such as multi-well plates, in order to readily facilitate implementation of the present methods. Enzymes that can be included in the present kits include nucleotide polymerases and the like. Solid supports can include beads and the like whereas molecular weight markers can include conjugatable markers, for example biotin and streptavidin or the like.

In one embodiment, the kit is made up of instructions for carrying out the method described herein for diagnosing a breast cancer from the luminal-B subtype in a female. The instructions can be provided in any intelligible form through a tangible medium, such as printed on paper, computer readable media, or the like.

Still a further aspect of the present invention refers to the use, for diagnosing a breast cancer from the luminal-B subtype in a female, of the abovementioned kit comprising at least one nucleic acid probe or oligonucleotide or at least one antibody, which can be used in a method as defined previously, for determining the copies number of the ZNF703 gene, and/or determining the expression of the ZNF703 gene.

In the following, the invention is described in more detail with reference to amino acid sequences, nucleic acid sequences and the examples. Yet, no limitation of the invention is intended by the details of the examples. Rather, the invention pertains to any embodiment which comprises details which are not explicitly mentioned in the examples herein, but which the skilled person finds without undue effort.

### Examples

### 1) 8p12 amplification is a recurrent copy number abnormality in luminal B tumors

To identify molecular mechanisms and signaling pathways specifically deregulated in luminal B tumors, we compared the genomic profiles from of luminal B and luminal A cases by using high density aCGH.

More especially, the tumor tissues were initially collected from 266 patients with invasive adenocarcinoma who underwent initial surgery at the Institute Paoli-Calmettes between 1987 and 2007, and from 22 samples from the Jules Bordet Institute. These samples were macrodissected and frozen in liquid nitrogen within 30 minutes of removal. DNA and RNA were extracted from frozen tumor samples by using guanidium isothiocynanate and cesium chloride gradient and their integrity was controlled on AGILENT BIOANALYZER (AGILENT TECHNOLOGIES) and agarose gel electrophoresis, respectively.

The genome profiling was then established on a subset of 100 luminal breast tumors (59 luminal A and 41 luminal B) for which RNA and DNA samples were both available. Because luminal B tumors have been associated with the amplifier genomic profile, we focused on recurrent amplifications. Genomic imbalances of 100 breast tumors were analyzed by aCGH using 244K CGH Microarrays (Hu-244A, AGILENT TECHNOLOGIES) according to the manufacturer's instruction. The final data set contained 225,388 unique probes covering 22,509 genes and intergenic part following the hg17 human genome mapping.

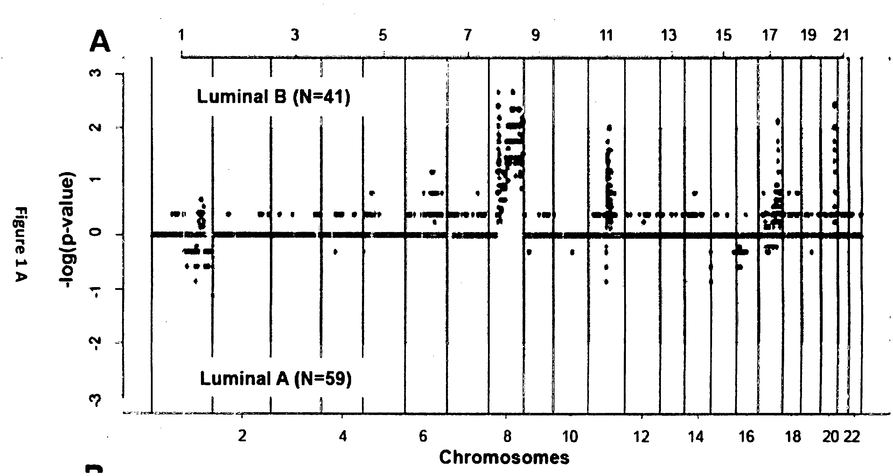

The figure 1A shows the results from the supervised analyses from the aCGH data with the genomic segments ordered on the X axis from chromosome 1 to 22 and on the Y axis according to their association with luminal A or B molecular subtype (-log(^{p-value})). The gray zone contains genomic segments that are not significantly associated with any of the two molecular subtypes (p>0.01) and over this gray zone is located genomic segments significantly amplified in luminal B breast tumors (p<0.01).

The results show that five genomic regions of recurrent high-level amplification (8p12, 8q22, 11q13, 17q24, 20q13) are associated with luminal B tumors (Fig. 1A; Fisher's exact test, p<0.01). In contrast, we did not identify any genomic region specifically amplified in luminal A tumors; this was not surprising since luminal A tumors have been associated with the simplex phenotype.

### 2) Luminal B tumors exhibit ZNF703 gene amplification and overexpression

We found a strong association of 8p12 amplification with luminal B tumors with 32% of tumors amplified; only 8% of luminal A tumors showed the 8p12 amplification, which was below the threshold retained by the aCGH statistical analysis (See Fig. 1A).

We searched to identify the 8p11-12 genes targeted by amplification by comparing for each gene, the association between copy number and mRNA expression level for the 38 genes comprised in the amplicon defined by the aCGH analysis. These genes are localized in a 4.6 Mb region spanning between *ZNF703* and *ANK1.*

For this analysis, the expression profiles of tumor samples wee obtained using AFFYMETRIX U133 Plus 2.0 human oligonucleotide microarrays and scanning was done with AFFYMETRIX GENEARRAY scanner. The data were analyzed by 'Robust Multichip Average' (RMA) with the non-parametric quantile algorithm as normalization parameter in R/Bioconductor and associated packages.

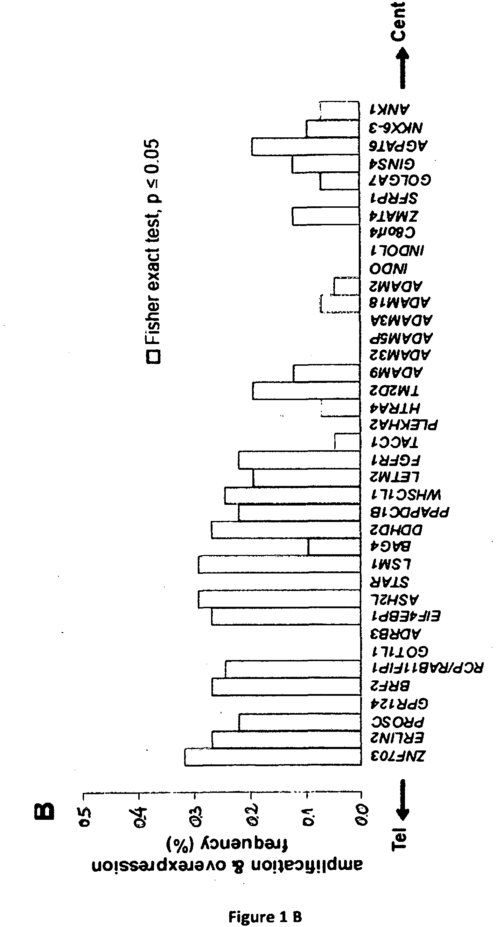

The figure 1B shows the combined genomic and transcriptional analysis for each gene from the 8p12 amplicon identified in luminal B breast tumors. Genes are ordered from telomere (left) to centromere (right) and the frequency of luminal B tumors presenting an increase in gene copy number and mRNA level is represented. Gray bars label genes that present a statistically significant association between gene amplification and overexpression (Fisher's exact test, p-value≤0.05).

The results identify a hot spot of amplification frequency for the genomic segment that contains only the *ZNF703* gene (Fisher's exact test, p<0.01) (data not shown). The results established that nineteen genes showed amplification correlated with upregulation (Fisher's exact test, p<0.01) (See Fig. 1B). Among them, *ZNF703* was the most frequently amplified and overexpressed gene.

In 11 breast cancer cell lines (BCL), we did a similar integrated analysis of genomic amplification (using this time quantitative PCR) and gene overexpression by both DNA microarrays and reverse-transcribed PCR for each of the 38 genes. These BCLs are derived from normal or malignant epithelium and include 184B5, CAMA-1, HCC1500, HME-1, MCF7, MCF10A, MDA-MB-134, T47D, ZR-75-1, SUM52 and S68. BCLs were grown using the recommended culture conditions. The DNA and RNA form BCLs were isolated utilizing RNEASY MINI KIT (QIAGEN) and used for QPCR and RTQPCR in a LightCycler FastStart DNA Master^{plus} SYBR Green I (ROCHE) according to manufacturers' instruction.

In BCLs, the results have shown that *ZNF703* expression levels measured by DNA microarrays and by quantitative RT-PCR were correlated (Pearson test, r=0.96), and were correlated with an increase in protein expression level (data not shown). Thus the integrated analysis on cell lines confirmed the results obtained on luminal B primary tumors, with *ZNF703* gene presenting the most significant correlation between DNA copy number and mRNA expression (Pearson test, r=0.9) (data not shown).

We next examined *ZNF703* expression measured by DNA microarrays according to the distribution of molecular subtypes distribution in 1172 breast cancers from 11 different published datasets.

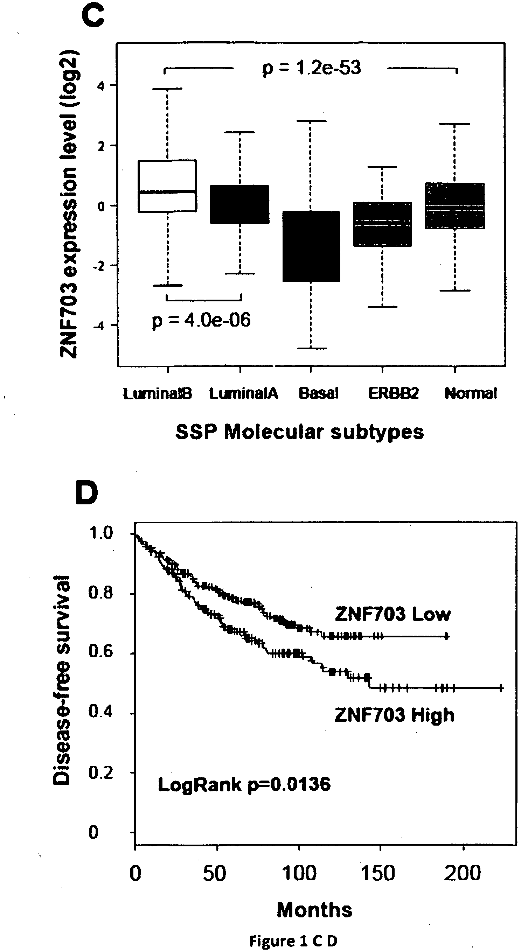

The figure 1C represents *ZNF703* expression distribution according to molecular subtypes of 1172 breast tumors.

The results confirmed that *ZNF703* overexpression was associated with luminal B tumors when compared with luminal A tumors (T test, p=4.10⁻⁶) or with other subtypes (ANOVA, p=1.2⁻⁵³) (See Fig. 1C).

To determine whether *ZNF703* overexpression had clinical impact, we looked for association with clinical and pathologic features in 561 luminal tumors (A and B).

The figure 1D shows the Kaplan-Meier survival curves according to *ZNF703* gene expression status.

The results have shown no correlation between *ZNF703* overexpression and any histoclinical factor. However, a high level of *ZNF703* mRNA was associated with a decrease in disease-free survival (DFS) with a 5-years DFS of 68% versus 78% for a low level of *ZNF703* (p=0.0136) (see Fig. 1D).

Taken together our results identified *ZNF703* as a plausible oncogene candidate.

### 3) Estrogen receptor regulates ZNF703 transcription

Some studies have described *ZNF703* as a target gene of ER transcriptional activity, which is the master transcriptional regulator of luminal tumor phenotype, and have identified estrogen response elements (ERE) in its promoter region.

To firmly establish that *ZNF703* expression is under the control of ER transcriptional activity, we cultured MDA-MB-134 and HCC1500 breast cancer cell lines in medium depleted or supplemented in 17β-estradiol (E2).

The results have shown that, in these cells, ZNF703 transcription and protein production was induced by addition of E2 in a time-dependent manner (data not shown).

Consequently, *ZNF703* is a potential oncogene under the control of ER activity in luminal B tumors.

### 4) Identification and characterization of ZNF703 in the nucleus

To identify signaling pathways regulated by ZNF703, ZNF703 was overexpressed in MCF7, a luminal breast cancer cell line that does not present 8p12 amplification, utilizing a GFP-ZNF703 fusion protein expression. Human *ZNF703* cDNA full length (IMAGE: 5527569) was obtained by. cDNA sequence was PCR-amplified using attB1-TCACCATGAGCGATTCGCCCGCTGG-3' (SEQ ID n°3) and attB2-CCTGGTATCCCAGCGCCGAGG-5' (SEQ ID n°4) oligonucleotides. The resulting PCR fragment was inserted into the vector pDONR™/Zeo using BP-reaction gateway technology (INVITROGEN). pDONR™/Zeo-ZNF703 was then subcloned in pEGFP-C1-DEST using LR-reaction (INVITROGEN). As control a pEGFP-C1 vector that produces only a GFP protein was used. MCF7 cells were transfected with pEGFP-ZNF703 or pEGFP-C1 by electroporation as described by the manufacturer (AMAXA). Individual clones stably expressing GFP-ZNF703 fusion protein or GFP were obtained by constant geneticin selection at 0.5 mg/ml, the resulting clones were confirmed by Western blotting.

For immunofluorescence, the MCF7 GFP and GFP-ZNF7O3 cells were grown on LABTEK chambers. After 48 hours, cells were fixed 20 minutes in 3.7% paraformaldehyde, PBS; permeabilized 5 minutes with PBS, 0.1% TRITON X-100®; blocked with protein block serum-free (DAKO) ; and incubated 1 hour with primary antibodies (anti-GFP (ROCHE, 1:250)), and with corresponding secondary antibody Alexa Fluor 594 (INVITROGEN) conjugated for 20 minutes incubation. Images of the nuclei were counterstained with DAPI/antifade (INVITROGEN) and coverslipped. Sections were examined with a fluorescent microscope (LEICA).

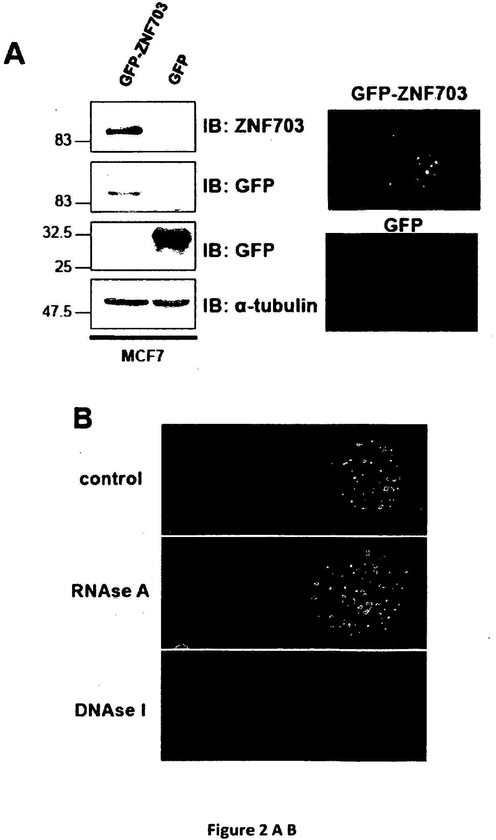

The figure 2A shows immunoblotting and immunofluorescence analyses of MCF7 GFP-ZNF7O3 and MCF7 GFP breast cancer cell lines. Immunoblotting with anti-ZNF703 and anti-GFP antibodies identifies GFP-ZNF703 fusion protein with a molecular weight of 83 kDa. α-tubulin protein expression was used as a control.

The Immunofluorescence results reveal nuclear dot-like structures in MCF7 GFP-ZNF703 cells whereas GFP was detected in all subcellular structures in MCF7 GFP cells. To avoid potential artefactual subcellular location of ZNF703 due to the fusion protein product with GFP, we confirmed that endogenous ZNF703 is present in the same structure (data not shown). The size and the morphology of these nuclear structures are reminiscent of promyelocytic leukemia (PML) oncogenic domains (PODs) and RNA splicing bodies (SBs), which contain different sets of proteins.

To examine the potential overlap of ZNF703 localization with these nuclear structures, MCF7 GFP-ZNF703 cells were subjected to immunohistochemistry with antibody against PML (SANTA-CRUZ, 1:20) as described previously and to DNase 1 (100 µg/ml) or RNase A (80 µg/ml) treatment for 30min and 15min respectively. The nuclei were counterstained with DAPI/antifade (INVITROGEN) and coverslipped. Sections were examined with a fluorescent microscope (LEICA).

The figure 2B shows the immunofluorescence obtained for MCF7 GFP-ZNF7O3 cells non-treated (control) or treated with RNAse A or DNAse I.

The results established that the staining pattern of PML did not overlap with that of ZNF703 (data not shown). The RNase A treatment did not affect ZNF703 nuclear structures (Fig. 2B) suggesting that ZNF703 bodies are distinct from both PODs and SBs. In contrast, DNase I treatment completely disorganized ZNF703 nuclear localization (Fig. 2B). Although ZNF703 protein has only one zinc finger motif, proscribing a direct DNA-biding, these results suggest that ZNF703 is part of nuclear structures that directly interact with DNA.

### 5) Identification of ZNF7O3 interactors

We next sought to identify proteins that associate with ZNF703 in the nucleus. This identification was done by immunoprecipitated with anti-GFP antibody (IP GFP) or beads only (No Ab), separated by SDS-PAGE, and stained with colloidal Coomassie blue. The copreciptated proteins were then identified by mass spectrometry as HSP60 (accession number, NP 955472); DCAF7 (NP 005819) and PHB2 (NP 001138303), respectively.

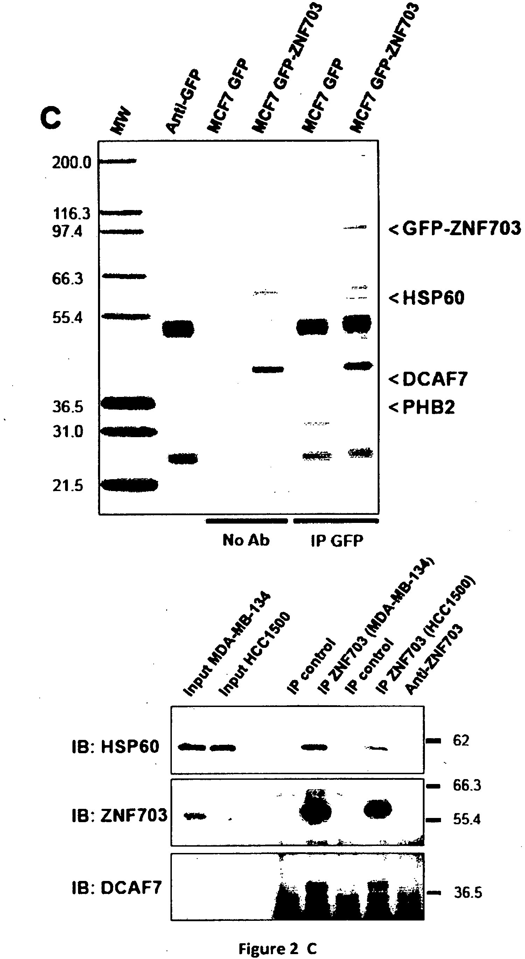

The Figure 2C shows the SDS-PAGE gel with the lane on the left corresponding to molecular weight standards (MW). The interaction between ZNF703 and HSP60 or DCAF7 was confirmed by ZNF7O3-immunoprecipitation, followed by Western blot analysis using anti-HSP60 or anti-DCAF7 antibody. Input represents whole cell extract from HCC1500 and MDA-MB-134.

Co-immunoprecipitation experiments confirmed the interaction of ZNF703 with HSP60 and DCAF7 in HCC1500 and MDA-MB-134 (endogenous conditions) and in MCF7 GFP-ZNF703 (Fig. 2C).

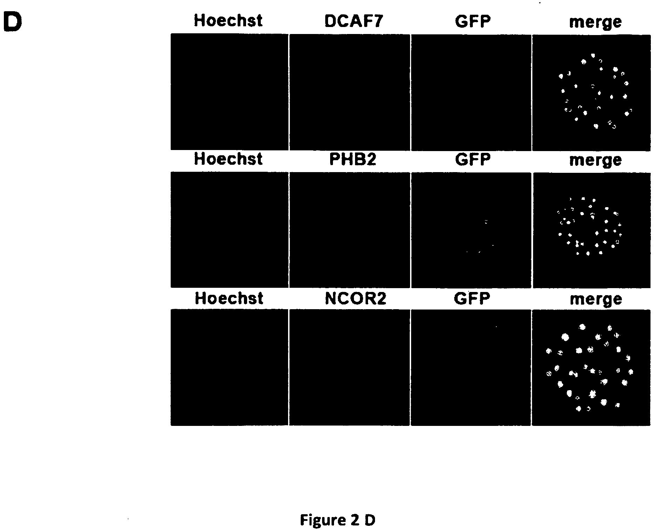

The figure 2D shows MCF7 cells overexpressing GFP-ZNF703 immunostained with anti-GFP, anti-DCAF7, anti-PHB2 or anti-SMRT/NCOR2 antibody. Nuclei are counterstained with Hoechst (blue).

The results show that DCAF7 and PHB2 colocalized with ZNF703 in the dot-like nuclear structures (Fig. 2D), whereas despite their interaction, ZNF703 and HSP60 did not colocalize in the nuclear matrix. HSP60 was only present in the cytoplasm and may interact with the cytoplasmic form of ZNF703 (data not shown) and ZNF703-HSP60 protein complex may have a distinct function from that of the ZNF703-DCAF7-PHB2 complex. PHB2 (also called REA for repressor of estrogen receptor activity) is a nuclear corepressor of ER activity in mammary gland development. Prohibitin mediates transcriptional repression through recruitment of additional nuclear corepressors (NCOR1/NCOR2) and HDACs. The staining pattern of NCOR2 overlapped completely with the ZNF703 nuclear structures detected by GFP fluorescence (Fig. 2D).

Interestingly, when we questioned the multi-experiment matrix (MEM) web interface for *ZNF703* gene expression similarities across 432 published gene expression datasets analyzed with AFFYMETRIX GENECHIP^{®} from cell lines and primary tumors studies, we identified *NCOR2* to be the most co-expressed gene with *ZNF703* (data not shown).

Altogether, our results suggest that nuclear ZNF703 is a cofactor of a nuclear corepressor complex and may contribute to transcriptional regulation.

### 6) ZNF703 overexpression increases cancer stem cell population

To further characterize ZNF703 overexpression effect, modification of gene expression program in MCF7 GFP-ZNF703 was analyzed compared to MCF7 GFP using DNA microarrays.

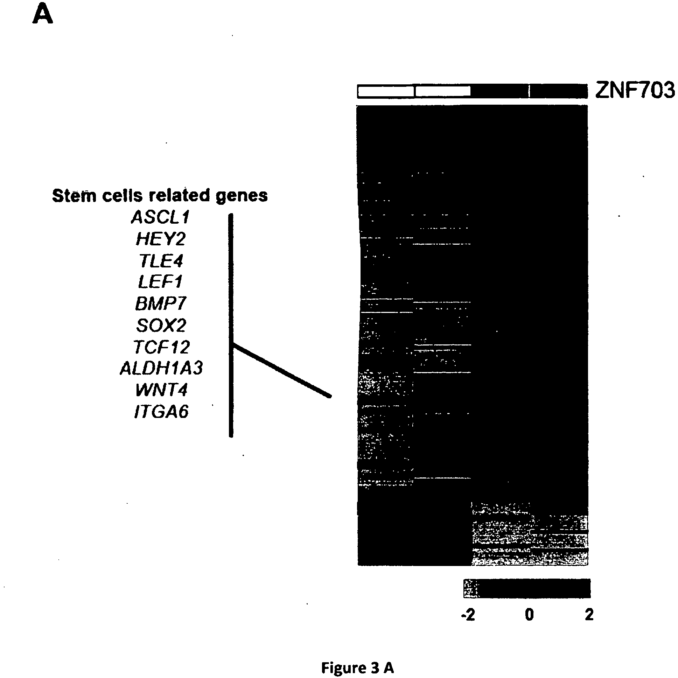

The figure 3A shows the gene expression profiles of two independent MCF7 GFP and two MCF7 GFP-ZNF703 stably expressing clones, wherein the 100 top genes regulated by ZNF703 overexpression are represented on hierarchical clustering. This gene list was highly enriched in stem cell-related genes.

A total of 189 genes were differentially expressed between the two cell lines with 160 genes induced and 29 genes downregulated by ZNF703 overexpression (Fig. 3A), which are disclosed in the following table 1.

**Table 1**

| Gene symbol | Locus | GeneID.MAJ | p-Value | Fold Change GFP-ZNF703 Vs GFP |
|---|---|---|---|---|
| IGFBP5 | 2q33-q36 | GeneID:3488 | 4,46752E-07 | 41,38 |
| ASCL1 | 12q23.2 | GeneID:429 | 3,26209E-06 | 37,71 |
| TOX3 | 16q12.1 | GeneID:27324 | 4,02801 E-09 | 32,96 |
| CLCA2 | 1p31-p22 | GeneID:9635 | 1,97621E-09 | 27,29 |
| FLJ25076 | 5p15.31 | GeneID:134111 | 2,49241 E-07 | 17,35 |
| GOLM1 | 9q21.33 | GeneID:51280 | 1,10303E-06 | 16,77 |
| LAMB1 | 7q22 | GeneID:3912 | 2,21971 E-06 | 11,75 |
| SCIN | 7p21.3 | GeneID:85477 | 8,14783E-06 | 11,35 |
| PKIA | 8q21.12 | GeneID:5569 | 3,01259E-06 | 10,86 |
| LDHB | 12p12.2-p12.1 | GeneID:3945 | 7,68319E-05 | 10,80 |
| KCNJ8 | 12p11.23 | GeneID:3764 | 3,08257E-06 | 10,76 |
| SLITRK6 | 13q31.1 | GeneID:84189 | 1,32896E-07 | 8,94 |
| SOCS2 | 12q | GeneID:8835 | 3,21882E-08 | 8,92 |
| PRSS23 | 11q14.1 | GeneID:11098 | 9,69909E-07 | 8,82 |
| CYBRD1 | 2q31.1 | GeneID:79901 | 6,67443E-07 | 8,04 |
| ADD3 | 10q24.2-q24.3 | GeneID:120 | 0,002466901 | 7,42 |
| ELF5 | 11p13-p12 | GeneID:2001 | 0,000318818 | 7,40 |
| TRGC2 | 7p14 | GeneID:6967 | 0,000734339 | 7,31 |
| LYN | 8q13 | GeneID:4067 | 7,32433E-05 | 7,10 |
| FHL1 | Xq26 | GeneID:2273 | 6,46899E-05 | 6,44 |
| CAV2 | 7q31.1 | GeneID:858 | 2,61328E-06 | 6,35 |
| MID1 | Xp22 | GeneID:4281 | 0,003784942 | 6,21 |
| LAMP3 | 3q26.3-q27 | GeneID:27074 | 0,000652264 | 6,09 |
| DOCK11 | Xq24 | GeneID:139818 | 3,19238E-05 | 6,04 |
| PLS3 | Xq23 | GeneID:5358 | 0,033894651 | 6,01 |
| HEY2 | 6q21 | GeneID:23493 | 6,0341E-05 | 5,90 |
| SLC1A1 | 9p24 | GeneID:6505 | 1,35557E-05 | 5,88 |
| EHF | 11p12 | GeneID:26298 | 0,002284825 | 5,79 |
| PDE4B | 1p31 | GeneID:5142 | 0,000541506 | 5,64 |
| CYP4Z1 | 1p33 | GeneID:199974 | 0,036286952 | 5,53 |
| LRP12 | 8q22.2-q23.1 | GeneID:29967 | 7,97536E-05 | 5,43 |
| TLE4 | 9q21.31 | GeneID:7091 | 5,61761 E-05 | 5,38 |
| FAM129A | 1q25 | GeneID:116496 | 2,65944E-06 | 5,20 |
| CAV1 | 7q31.1 | GeneID:857 | 0,028238795 | 5,10 |
| WDR72 | 15q21.3 | GeneID:256764 | 0,00042777 | 5,09 |
| LEF1 | 4q23-q25 | GeneID:51176 | 0,000831685 | 5,07 |
| IGFBP3 | 7p13-p12 | GeneID:3486 | 7,39501 E-05 | 5,00 |
| MARCKS | 6q22.2 | GeneID:4082 | 0,000417374 | 4,82 |
| CHN2 | 7p15.3 | GeneID:1124 | 0,001381717 | 4,72 |
| B3GNT5 | 3q28 | GeneID:84002 | 2,47084E-05 | 4,71 |
| RAP1A | 1p13.3 | GeneID:5906 | 0,006331496 | 4,62 |
| STEAP4 | 7q21.12 | GeneID:79689 | 0,00307879 | 4,55 |
| BMP7 | 20q13 | GeneID:655 | 1,81653E-08 | 4,51 |
| FABP5 | 8q21.13 | GeneID:2171 | 0,003828884 | 4,51 |
| SASH1 | 6q24.3 | GeneID:23328 | 5,54456E-06 | 4,43 |
| GALNT12 | 9q22.33 | GeneID:79695 | 0,003735222 | 4,39 |
| TCF12 | 15q21 | GeneID:6938 | 0,019599851 | 4,36 |
| AOX1 | 2q33 | GeneID:316 | 0,000215804 | 4,35 |
| UGT1A10 | 2q37 | GeneID:54575 | 0,034831355 | 4,24 |
| KCTD12 | 13q22.3 | GeneID:115207 | 0,00275157 | 4,23 |
| GP2 | 16p12 | GeneID:2813 | 0,000158843 | 4,13 |
| PKIB | 6q22.31 | GeneID:5570 | 0,018773343 | 4,11 |
| SOX2 | 3q26.3-q27 | GeneID:6657 | 0,001182563 | 3,91 |
| LMO3 | 12p12.3 | GeneID:55885 | 0,000702554 | 3,85 |
| MUCL1 | 12q | GeneID:118430 | 0,001468657 | 3,75 |
| MUM1L1 | Xq22.3 | GeneID:139221 | 0,016761537 | 3,72 |
| TMSL8 | Xq21.33-q22.3 | GeneID:11013 | 0,002251086 | 3,65 |
| FGF13 | Xq26.3 | GeneID:2258 | 0,001837133 | 3,63 |
| SGCE | 7q21-q22 | GeneID:8910 | 0,044442871 | 3,63 |
| GOLSYN | 8q23.2 | GeneID:55638 | 0,002059708 | 3,61 |
| CALML5 | 10p15.1 | GeneID:51806 | 0,015517029 | 3,35 |
| JAZF1 | 7p15.2-p15.1 | GeneID:221895 | 0,016866115 | 3,31 |
| ANKH | 5p15.1 | GeneID:56172 | 6,34963E-05 | 3,30 |
| ATP8A2 | 13q12 | GeneID:51761 | 0,014550989 | 3,28 |
| ALDH1A3 | 15q26.3 | GeneID:220 | 0,018233899 | 3,27 |
| BHLHB3 | 12p11.23-p12.1 | GeneID:79365 | 8,66133E-06 | 3,27 |
| RIN2 | 20p11.22 | GeneID:54453 | 0,006801811 | 3,27 |
| GDPD1 | 17q22 | GeneID:284161 | 0,028173812 | 3,26 |
| NMU | 4q12 | GeneID:10874 | 3,13485E-07 | 3,25 |
| MARCH8 | 10q11.21 | GeneID:220972 | 0,014191813 | 3,24 |
| KLK10 | 19q13.3-q13.4 | GeneID:5655 | 0,012655822 | 3,21 |
| TFF3 | 21q22.3 | GeneID:7033 | 8,24186E-08 | 3,19 |
| CREB3L1 | 11p11.2 | GeneID:90993 | 0,008961066 | 3,16 |
| C14orf132 | 14q32.2 | GeneID:56967 | 0,000224584 | 3,15 |
| CXADR | 21q21.1 | GeneID:1525 | 5,7832E-06 | 3,15 |
| NUPR1 | 16p11.2 | GeneID:26471 | 8,70831E-05 | 3,12 |
| WNT4 | 1 p36.23-p35.1 | GeneID:54361 | 0,010082731 | 3,12 |
| UGT1A4 | 2q37 | GeneID:54657 | 0,031127338 | 3,11 |
| GRHL3 | 1p36.11 | GeneID:57822 | 0,022305377 | 3,08 |
| GALNTL4 | 11p15.3 | GeneID:374378 | 0,007233079 | 3,08 |
| PDE4D | 5q12 | GeneID:5144 | 0,006054849 | 2,99 |
| CXCR4 | 2q21 | GeneID:7852 | 0,001059867 | 2,99 |
| ITGA6 | 2q31.1 | GeneID:3655 | 0,008246878 | 2,99 |
| CRYBG3 | 3q11.2 | GeneID:131544 | 0,019038988 | 2,92 |
| SYTL4 | Xq21.33 | GeneID:94121 | 0,007063578 | 2,83 |
| PCDH9 | 13q14.3-q21.1 | GeneID:5101 | 8,88445E-05 | 2,82 |
| BDKRB2 | 14q32.1-q32.2 | GeneID:624 | 0,021356215 | 2,79 |
| FN1 | 2q34 | GeneID:2335 | 0,008339693 | 2,78 |
| PTGER4 | 5p13.1 | GeneID:5734 | 0,002015333 | 2,77 |
| DSC2 | 18q12.1 | GeneID:1824 | 0,0217683 | 2,77 |
| NTN1 | 17p13-p12 | GeneID:9423 | 0,000671193 | 2,75 |
| SUSD4 | 1q41 | GeneID:55061 | 5,19031E-05 | 2,75 |
| PPAP2A | 5q11 | GeneID:8611 | 0,000618911 | 2,73 |
| KLK5 | 19q13.3-q13.4 | GeneID:25818 | 0,049352175 | 2,72 |
| SUSD2 | 22q11-q12 | GeneID:56241 | 0,000254485 | 2,72 |
| HERC5 | 4q22.1 | GeneID:51191 | 0,016370139 | 2,71 |
| RCAN1 | 21q22.1-q22.2121q 22.12 | GeneID:1827 | 0,000244694 | 2,71 |
| MET | 7q31 | GeneID:4233 | 0,018229157 | 2,65 |
| BTBD11 | 12q23.3 | GeneID:121551 | 0,010770389 | 2,65 |
| MMD | 17q | GeneID:23531 | 2,11974E-05 | 2,61 |
| PRICKLE1 | 12q12 | GeneID:144165 | 0,037201447 | 2,61 |
| UBE2L6 | 11q12 | GeneID:9246 | 0,000662903 | 2,60 |
| SLC27A2 | 15q21.2 | GeneID:11001 | 1,66074E-05 | 2,59 |
| XK | Xp21.1 | GeneID:7504 | 0,036299197 | 2,58 |
| MOBKL2B | 9p21.2 | GeneID:79817 | 0,031366534 | 2,57 |
| GRB14 | 2q22-q24 | GeneID:2888 | 0,000967898 | 2,57 |
| FAR2 | 12p11.22 | GeneID:55711 | 0,046422085 | 2,57 |
| TES | 7q31.2 | GeneID:26136 | 0,001059813 | 2,54 |
| PMEPA1 | 20q13.311 20q13.31-q13.33 | GeneID:56937 | 0,003157106 | 2,54 |
| LCN2 | 9q34 | GeneID:3934 | 0,027545791 | 2,54 |
| VLDLR | 9p24 | GeneID:7436 | 0,025403187 | 2,53 |
| DIAPH2 | Xq21.33 | GeneID:1730 | 0,033789035 | 2,51 |
| HUNK | 21q22.1 | GeneID:30811 | 0,048072151 | 2,51 |
| RNF144A | 2p25.2-p25.1 | GeneID:9781 | 0,047499753 | 2,48 |
| FAM84A | 2p24.3 | GeneID:151354 | 2,33258E-05 | 2,43 |
| PKP1 | 1q32 | GeneID:5317 | 0,030495823 | 2,40 |
| SDC2 | 8q22-q23 | GeneID:6383 | 0,01193805 | 2,40 |
| NPR3 | 5p14-p13 | GeneID:4883 | 0,024983984 | 2,38 |
| CSRP2 | 12q21.1 | GeneID:1466 | 0,000841294 | 2,38 |
| KAL1 | Xp22.32 | GeneID:3730 | 8,09439E-05 | 2,37 |
| RAI14 | 5p13.3-p13.2 | GeneID:26064 | 0,029515906 | 2,36 |
| ACPL2 | 3q23 | GeneID:92370 | 0,001277764 | 2,36 |
| DHRS3 | 1p36.1 | GeneID:9249 | 0,000431189 | 2,34 |
| PRIMA1 | 14q32.13 | GeneID:145270 | 0,047829804 | 2,31 |
| DKK1 | 10q11.2 | GeneID:22943 | 4,14822E-06 | 2,30 |
| BCAS1 | 20q13.2 | GeneID:8537 | 0,018290909 | 2,30 |
| VIM | 10p13 | GeneID:7431 | 0,000993006 | 2,28 |
| KLF13 | 15q12 | GeneID:51621 | 0,035241038 | 2,28 |
| C21orf63 | 21q22.11 | GeneID:59271 | 0,000517009 | 2,28 |
| CTSC | 11q14.1-q14.3 | GeneID:1075 | 0,004350675 | 2,27 |
| MNS1 | 15q21.3 | GeneID:55329 | 0,0027479 | 2,26 |
| CYFIP2 | 5q33.3 | GeneID:26999 | 6,38755E-06 | 2,22 |
| LXN | 3q25.32 | GeneID:56925 | 0,004646091 | 2,22 |
| RPS6KA3 | Xp22.2-p22.1 | GeneID:6197 | 5,3261 E-05 | 2,19 |
| KCNMA1 | 10q22.3 | GeneID:3778 | 0,002257739 | 2,19 |
| RCN1 | 11p13 | GeneID:5954 | 0,000960313 | 2,18 |
| LYPD1 | 2q21.2 | GeneID:116372 | 0,041568123 | 2,18 |
| PCP4 | 21q22.2 | GeneID:5121 | 0,023307837 | 2,17 |
| PLA2G4C | 19q13.3 | GeneID:8605 | 0,002894926 | 2,17 |
| HS6ST2 | Xq26.2 | GeneID:90161 | 0,015642484 | 2,16 |
| TIAM1 | 21q22.112 1q22.11 | GeneID:7074 | 0,000237626 | 2,16 |
| AMOTL1 | 11q14.3 | GeneID:154810 | 0,000598874 | 2,15 |
| ACOX2 | 3p14.3 | GeneID:8309 | 0,001973971 | 2,15 |
| EPB41 L4A | 5q22.2 | GeneID:64097 | 0,001139804 | 2,14 |
| C11orf75 | 11q13.3-q23.3 | GeneID:56935 | 0,000427264 | 2,14 |
| TMEM45B | 11q24.3 | GeneID:120224 | 0,000130871 | 2,14 |
| HIPK2 | 7q32-q34 | GeneID:28996 | 0,000256851 | 2,14 |
| HSD17B8 | 6p21.3 | GeneID:7923 | 0,001119851 | 2,13 |
| PARP9 | 3q13-q21 | GeneID:83666 | 0,001939111 | 2,12 |
| DDIT4 | 10pter-q26.12 | GeneID:54541 | 0,003520499 | 2,12 |
| TNS3 | 7p12.3 | GeneID:64759 | 0,000250908 | 2,10 |
| MORC4 | Xq22.3 | GeneID:79710 | 5,0853E-05 | 2,08 |
| CDK6 | 7q21-q22 | GeneID:1021 | 0,000884049 | 2,07 |
| TMPRSS4 | 11q23.3 | GeneID:56649 | 0,005044076 | 2,06 |
| BACE2 | 21q22.3 | GeneID:25825 | 0,001226238 | 2,05 |
| EPAS1 | 2p21-p16 | GeneID:2034 | 0,000677003 | 2,04 |
| FJX1 | 11p13 | GeneID:24147 | 0,013800638 | 2,03 |
| PRR15 | 7p15.1 | GeneID:222171 | 0,000607682 | 2,01 |
| SRI | 7q21.1 | GeneID:6717 | 0,034454376 | 2,00 |
| TGFB3 | 14q24 | GeneID:7043 | 0,007422388 | 2,00 |
| GFRA1 | 10q26 | GeneID:2674 | 0,000209607 | -2,01 |
| SLC16A1 | 1p12 | GeneID:6566 | 0,011836889 | -2,02 |
| COL12A1 | 6q12-q13 | GeneID:1303 | 0,000886768 | -2,03 |
| FAM26F | 6q22.1 | GeneID:441168 | 0,000369385 | -2,05 |
| KYNU | 2q22.2 | GeneID:8942 | 0,000204165 | -2,11 |
| PDP2 | 16q22.1 | GeneID:57546 | 0,000359386 | -2,11 |
| SYNGR1 | 22q13.1 | GeneID:9145 | 0,000951179 | -2,12 |
| RHOBTB3 | 5q15 | GeneID:22836 | 0,00042387 | -2,21 |
| STC1 | 8p21-p11.2 | GeneID:6781 | 0,00097735 | -2,27 |
| EPN2 | 17p11.2 | GeneID:22905 | 0,000953798 | -2,29 |
| NFKBIZ | 3p12-q12 | GeneID:64332 | 0,000712042 | -2,36 |
| PTGES | 9q34.3 | GeneID:9536 | 6,04162E-06 | -2,72 |
| KCNE4 | 2q36.3 | GeneID:23704 | 0,003360104 | -2,80 |
| TFPI | 2q32 | GeneID:7035 | 1,65527E-05 | -2,80 |
| NT5C3L | 17q21.2 | GeneID:115024 | 0,001204634 | -2,81 |
| PMP22 | 17p12-p11.2 | GeneID:5376 | 0,01721727 | -2,89 |
| APOBEC3B | 22q13.1-q13.2 | GeneID:9582 | 5,79497E-06 | -2,90 |
| C8orf57 | 8q21.3 | GeneID:84257 | 0,000277604 | -3,31 |
| HSPB8 | 12q24.23 | GeneID:26353 | 1,05348E-06 | -3,46 |
| C10orf112 | 10p12.33 | GeneID:340895 | 2,54951 E-07 | -3,75 |
| ADAMTS19 | 5q31 | GeneID:171019 | 0,030765204 | -4,30 |
| COL4A5 | Xq22 | GeneID:1287 | 0,02136181 | -4,53 |
| PUNC | 15q22.3-q23 | GeneID:9543 | 0,011963244 | -4,88 |
| MMP16 | 8q21 | GeneID:4325 | 0,010551402 | -5,08 |
| UGT8 | 4q26 | GeneID:7368 | 0,015317508 | -5,26 |
| NECAB1 | 8q21.3 | GeneID:64168 | 0,043679126 | -5,40 |
| KCNK2 | 1q41 | GeneID:3776 | 0,007840396 | -5.51 |
| SYNPO2 | 4q26 | GeneID:171024 | 0,008504598 | -5,53 |
| WIPF1 | 2q31.1 | GeneID:7456 | 0,002043182 | -8,12 |

Among the 160 genes induced by ZNF703, several genes were related to WNT (such as *LEF1, TCF12, WNT4*) or NOTCH (such as *ASCL1, HEY2, TLE4*) signaling pathways. Activation of these pathways is known to regulate self-renewal program in breast cancer stem cells (CSC). To better characterize the association between genes induced by ZNF703 and stem cell-related genes, we measured the enrichment of 26 published stem cell-related gene sets in genes differentially expressed in MCF7 GFP-ZNF703. Ten out of 26 gene sets were enriched in the ZNF703-related genes (data not shown).

These results suggest that ZNF703 overexpression may be implicated in the regulation of breast CSC. To directly test this hypothesis, we used an *in vitro* tumorsphere assay, a powerful surrogate method to evaluate cancer stem/progenitor cells. MCF7 GFP-ZNF707 and MCF7 GFP cell lines were plated as single cells in 1% agarose coated plates at a density of 1000 cells/ml and grown for 5 days. The number of primary spheres was counted under microscope. Subsequent cultures after dissociation of primary spheres were plated in 1% coated plates at a density of 1000 cells/ml. As for primary spheres, the number of secondary spheres was determined under microscope. Experiments were done in triplicate. Tumorsphere cultures were grown in a serum-free mammary epithelium basal medium as previously described (GINESTIER et al., Cell Cycle, vol.8, p:3297-3302, 2009).

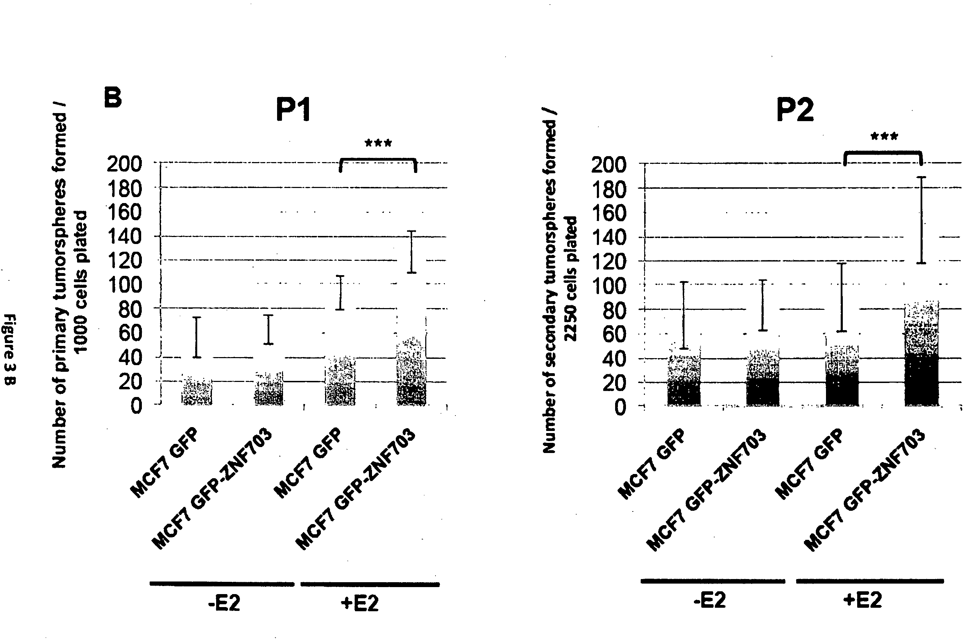

The figure 3B shows the tumorsphere formation for MCF7 GFP-ZNF703 and GFP cells in the presence or absence of 17-β estradiol (E2).

The results show that when stimulated by E2, MCF7 GFP-ZNF703 cells presented a significant increase in primary tumorsphere formation. Similar results were observed for secondary tumorsphere formation (Fig. 3B). These results suggest that ZNF703 overexpression increases the CSC population and may act as a regulator of self-renewal program in ER-positive cells.

### 7) ZNF703 overexpression regulates ER and E2F1 transcriptional activity

The above observations suggested that ZNF703 could regulate breast CSCs self-renewal activity by interacting with nuclear corepressor complex PHB2/NCOR2. Nuclear corepressors modulate the activity of master transcriptional regulators, such as ER or E2F1, and thus regulate key cell processes. Both ER and E2F1 have been implicated in the control of differentiation and self-renewal programs in stem cells. Therefore, we speculated that ZNF703 overexpression may regulate breast CSC biology through regulation of ER and E2F1 transcriptional activities. To test this hypothesis we first studied ER protein expression by immunostaining in MCF7 GFP-ZNF703.

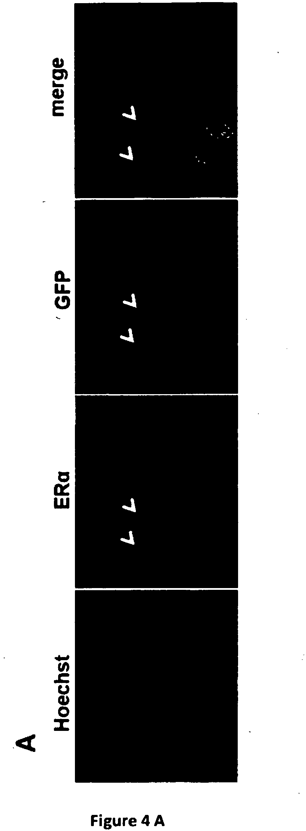

The figure 4A shows MCF7 GFP-ZNF703 cells were fixed and immunostained with anti-GFP and anti-ER antibodies and the nuclei counterstained with Hoechst.

The results show a decrease of ER expression in cells with ZNF703 dot-like structures (Fig. 4A).

Then, a specific luciferase reporter assay was used for the detection of estrogen transcriptional activity (p17M-ERE-Luc). MCF7 GFP and GFP-ZNF703 stable cell lines were electroporated (AMAXA) with 250 ng TK-R-Luc (pGL3: PROMEGA) as an internal control and 1µg of one luciferase reporter vector according to the manufacturer's protocol. For ER-related transcriptional activity, cells were seeded overnight in 60-mm dishes containing phenol red-free RPMI 1640 (INVITROGEN) supplemented with 10% charcoal dextrantreated fetal bovine serum (HYCLONE). 16 hours later, 10nM of 17-β estradiol (E2) were added to the medium and luciferase activity was determined after 24 hours. For luciferase activity detection, transfected cells lysate were prepared and THE DUAL-LUCIFERASE REPORTER ASSAY System was used (PROMEGA) according to the manufacturer's instructions. Each luciferase activity value was then normalized by protein concentration determined by Bradford assay and TK-renilla luciferase activity value. Each experiment was done in duplicate.

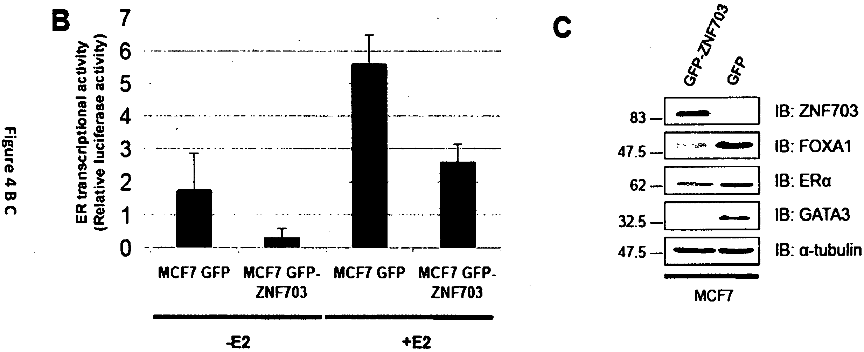

The figure 4B shows the evaluation of estrogen receptor-dependent transcriptional activity using a luciferase reporter system (p17M-ERE-Luc) in the presence and absence of 17-β estradiol (E2) in MCF7 GFP and GFP-ZNF703 breast cancer cell lines.

The results show a decrease of ER transcriptional activity in MCF7 GFP-ZNF703 compared to MCF7 GFP cells, independently of E2 induction (see Fig. 4B).

The figure 4C show the subsequent analysis of ER, FOXA1, and GATA3 protein expression by immunoblotting in MCF7 GFP-ZNF703 and MCF7 GFP breast cancer cell lines.

The results show then that MCF7 GFP-ZNF703 presented a decrease in expression for the two main ER-associated proteins. FOXA1 and GATA3 (See Fig. 4C) and a lower ER□ expression comparatively to the MCF7 GFP. These results indicate that ZNF703 modulates ER transcriptional activity and may control breast CSC differentiation process through this regulatory mechanism.

The effect of ZNF703 overexpression on the other classical nuclear target of corepressors, E2F1 was next examined.

Two different E2F1 reporter systems (p3XE2F1-Luc and pCCNE-Luc) was then used according to the luciferase assay protocol described previously. Both vectors code for the luciferase protein either under the control of three E2F1 responsive elements or under the control of Cyclin E (CCNE) promoter, a direct E2F1 target.

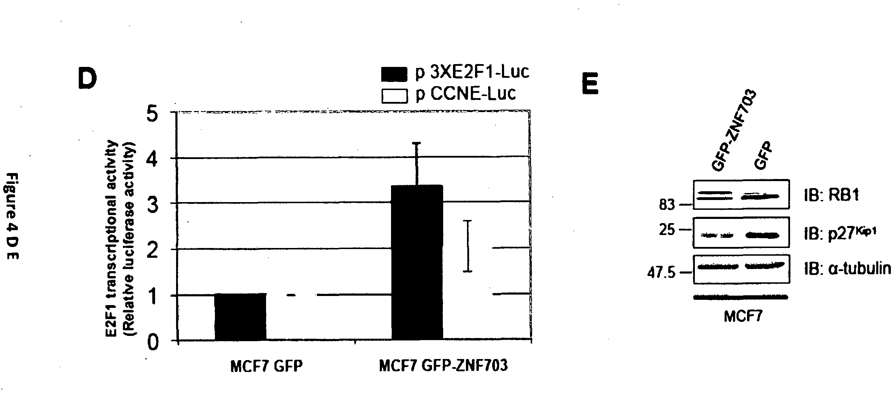

The figure 4D shows the evaluation of these two different luciferase reporter systems.

Both reporter assays show an increase of E2F1 transcriptional activity induced by ZNF703 overexpression (See Fig. 4D).

We further tested the functional activation of E2F1 by western blot analysis of RB1 and P27^{kip1} protein status. Both proteins are implicated in E2F1 regulation. RB1 binds E2F1 and represses cell cycle progression; phosphorylations inactivate RB1 that releases E2F1 leading to proteolysis of P27^{kip1} and to transcription of several target genes implicated in cell cycle progression from G1 to S.

The figure 4E then show the analysis of RB1 and P27^{kip1} protein expression by immunoblotting in MCF7 GFP-ZNF703 and MCF7 GFP cells with α-tubulin protein expression used as a control.

The results show an increase of RB1 phosphorylation and a decrease of P27^{kip1} protein expression in MCF7 GFP-ZNF703 attesting of E2F1 activation induced by ZNF703 overexpression (See Fig. 4E). It is interesting to note that the PHB1/NCOR1 complex is known to repress E2F1 transcriptional activity, whereas ZNF703/PHB2/NCOR2 complex seems to positively regulate E2F1 activity.

Altogether, these results suggest that ZNF703 may be implicated in the regulation of ER and E2F1 transcriptional regulators and thus may modulate gene expression program implicated in breast CSC biology through these two major pathways.

### 8) Luminal B tumors present an activation of E2F1 transcriptional program and a decrease in the activity of ER-related transcription factors

To extend these results to clinical samples of luminal B primary tumors, the Gene Set Enrichment Analysis (GSEA) {http://www.broadinstitute.org/gsea/} algorithm was used to screen the activated transcription factors from the Broad Institute (MSigDB C3: motif gene sets TFT) for identifying *a priori* defined sets of genes that were differentially expressed between luminal A and luminal B samples. GSEA was applied on the IPC series with 138 luminal BCs (i.e. the 49 luminal 8 and 89 luminal A). Prior the GSEA, data were filtered to remove low and poorly measured expression probe sets as defined by an expression value inferior to 6.64 log2 units across the 138 BCs. GSEA was done using signal-to-noise metric for ranking genes, weighted enrichment statistic to computed enrichment score (ES) of each gene sets tested and 1000 phenotype permutations to evaluate significance. As gene sets database, we chose the transcription factor targets list (C3, TFT) from the Molecular Signatures database (http://www.brondinstitute.org/gsea/msigdb). The C3 TFT list contains gene sets that share a transcription factor-binding site defined in the TRANSFAC database {http://www.gene-regulation.com}. Gene sets were defined as significant at the 5% level with a False Discovery Rate (FDR) under 25%. Relation between each transcription factor and E2F1 or ESR1/ERa was established by mining PubMed abstracts using Chilibot.

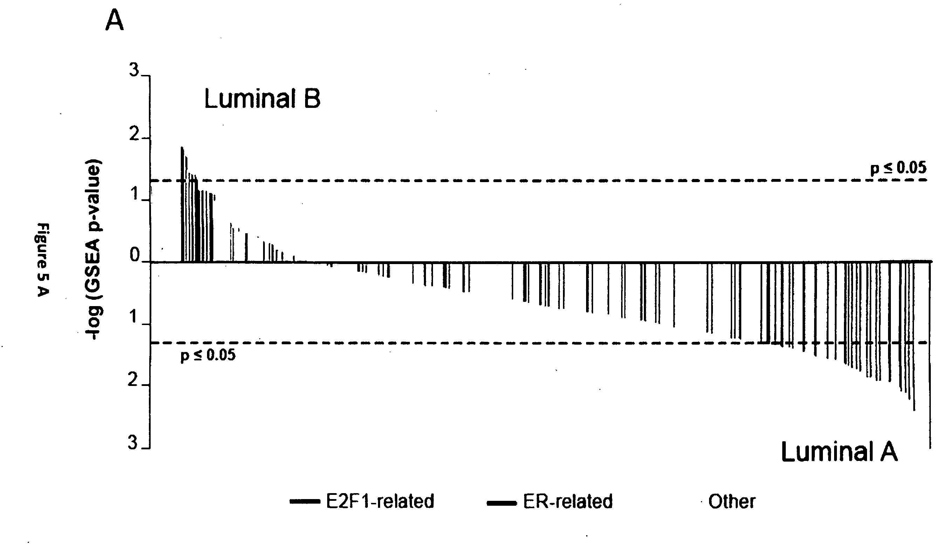

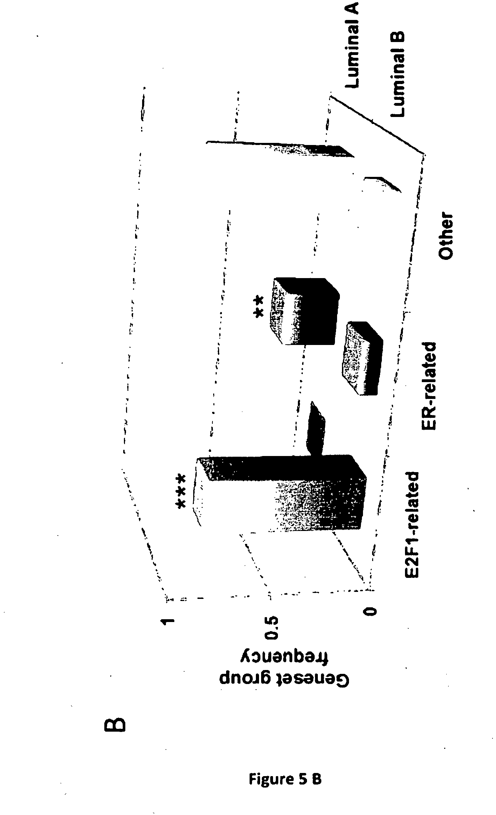

The figures 5A and 5B represent the different gene sets that share a transcription factor binding site defined in the TRANSFAC database enriched in luminal B tumors compared to luminal A tumors. Each gene sets is plotted according to its association to either luminal A or B tumors (-log(diff GSEA p-value)).

The results show a total of 119 gene sets that were differentially regulated between luminal B and luminal A tumors (FDR q-val<0.25: Nominal p-value<0.05) (See Fig. 5A). Among these gene sets, twelve were associated with luminal B tumors and 107 with luminal A tumors. Eight out of the eleven gene sets (72%) associated with luminal B tumors were related to genes containing E2F1 cis-regulatory motifs. Among the 107 gene sets associated with luminal A tumors, 31 (29%) were related to genes containing EREs or to ER-associated transcription factors whereas none of these gene sets was related to E2F1 transcription factor (See Fig. 5B). These results are consistent with our results suggesting that ZNF703 is a specific luminal B oncogene regulating E2F1 and ER transcriptional activity. It seems that in luminal B cancer cells the balance between E2F1 and ER activation is tilted toward E2F1 transcriptional program, in which ZNF703 may act as a key modulator.

## Claims

1. An *in vitro* method for diagnosing a breast cancer of the luminal-B subtype in a female, which comprises the steps of analyzing a biological sample from said female by:
i) determining the copy number of the Zinc Finger Protein 703 gene (ZNF703 gene), and/or
ii) determining the expression of the ZNF703 gene
wherein an increased copy number and/or an over-expression of the ZNF703 gene is indicative of a luminal B tumor.

2. The method of claim 1, wherein said luminal B tumor is associated with poor prognosis.

3. The method of claim 1, wherein an increased copy number and/or an over-expression of the ZNF703 gene is indicative of an increase of the Cancer Stem Cells (CSC) population.

4. The method of claim 1, wherein anti-estrogens are not selected as a treatment for the female diagnosed with breast cancer of the luminal-B subtype but instead more aggressive treatments such as chemotherapy are selected for the female diagnosed with breast cancer of the luminal-B subtype

5. The method of claim 1, wherein the female is a woman.

6. The method of claim 1, wherein said female is thought to develop or developing a breast cancer.

7. The method of claim 1, wherein the biological sample is a breast tissue sample.

8. The method of claim 1, wherein the biological sample is a breast tumor sample.

9. The method of claim 1, wherein said method comprises the step of determining the copy number of the ZNF703 gene.

10. The method of claim 1, wherein said increased copy number of the ZNF703 gene refers to a copy number of the ZNF703 gene by genome, which is superior to the two alleles of the ZNF703 gene.

11. The method of claim 1, wherein said method comprises the step of determining the expression of the ZNF703 gene.

12. The method of claim 11, wherein said determining step is assessed by analyzing the expression of mRNA transcript or mRNA precursors of said gene.

13. The method of claim 11, wherein said determining step is assessed by analyzing the expression of the ZNF703 protein translated from said gene.

14. An in vitro method for diagnosing a breast cancer of the luminal-B subtype in a female, which comprises the steps of analyzing a biological sample from said female by determining the expression of the ZNF703 gene, wherein an over-expression of the ZNF703 gene is indicative of a luminal B tumor and wherein said determining step is assessed indirectly by determining the expression of at least 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185 or 189 of the 189 genes listed in table 1:
| Gene symbol | GeneID.MAJ | | MID1 | GeneID:4281 |
|---|---|---|---|---|
| IGFBP5 | GeneID:3488 | | LAMP3 | GeneID:27074 |
| ASCL1 | GeneID:429 | | DOCK11 | GeneID:139818 |
| TOX3 | GeneID:27324 | | PLS3 | GeneID:5358 |
| CLCA2 | GeneID:9635 | | HEY2 | GeneID:23493 |
| FLJ25076 | GeneID:134111 | | SLC1A1 | GeneID:6505 |
| GOLM1 | GeneID:51280 | | EHF | GeneID:26298 |
| LAMB1 | GeneID:3912 | | PDE4B | GeneID:5142 |
| SCIN | GeneID:85477 | | CYP4Z1 | GeneID:199974 |
| PKIA | GeneID:5569 | | LRP12 | GeneID:29967 |
| LDHB | GeneID:3945 | | TLE4 | GeneID:7091 |
| KCNJ8 | GeneID:3764 | | FAM129A | GeneID:116496 |
| SLITRK6 | GeneID:84189 | | CAV1 | GeneID:857 |
| SOCS2 | GeneID:8835 | | WDR72 | GeneID:256764 |
| PRSS23 | GeneID:11098 | | LEF1 | GeneID:51176 |
| CYBRD1 | GeneID:79901 | | IGFBP3 | GeneID:3486 |
| ADD3 | GeneID:120 | | MARCKS | GeneID:4082 |
| ELF5 | GeneID:2001 | | CHN2 | GeneID:1124 |
| TRGC2 | GeneID:6967 | | B3GNT5 | GeneID:84002 |
| LYN | GeneID:4067 | | RAP1A | GeneID:5906 |
| FHL1 | GeneID:2273 | | STEAP4 | GeneID:79689 |
| CAV2 | GeneID:858 | | BMP7 | GeneID:655 |
| FABP5 | GeneID:2171 | | BDKRB2 | GeneID:624 |
| SASH1 | GeneID:23328 | | FN1 | GeneID:2335 |
| GALNT12 | GeneID:79695 | | PTGER4 | GeneID:5734 |
| TCF12 | GeneID:6938 | | DSC2 | GeneID:1824 |
| AOX1 | GeneID:316 | | NTN1 | GeneID:9423 |
| UGT1A10 | GeneID:54575 | | SUSD4 | GeneID:55061 |
| KCTD12 | GeneID:115207 | | PPAP2A | GeneID:8611 |
| GP2 | GeneID:2813 | | KLK5 | GeneID:25818 |
| PKIB | GeneID:5570 | | SUSD2 | GeneID:56241 |
| SOX2 | GeneID:6657 | | HERC5 | GeneID:51191 |
| LMO3 | GeneID:55885 | | RCAN1 | GeneID:1827 |
| MUCL1 | GeneID:118430 | | MET | GeneID:4233 |
| MUM1L1 | GeneID:139221 | | BTBD11 | GeneID:121551 |
| TMSL8 | GeneID:11013 | | MMD | GeneID:23531 |
| FGF13 | GeneID:2258 | | PRICKLE1 | GeneID: 144165 |
| SGCE | GeneID:8910 | | UBE2L6 | GeneID:9246 |
| GOLSYN | GeneID:55638 | | SLC27A2 | GeneID:11001 |
| CALML5 | GeneID:51806 | | XK | GeneID:7504 |
| JAZF1 | GeneID:221895 | | MOBKL2B | GeneID:79817 |
| ANKH | GeneID:56172 | | GRB14 | GeneID:2888 |
| ATP8A2 | GeneID:51761 | | FAR2 | GeneID:55711 |
| ALDH1A3 | GeneID:220 | | TES | GeneID:26136 |
| BHLHB3 | GeneID:79365 | | PMEPA1 | GeneID:56937 |
| RIN2 | GeneID:54453 | | LCN2 | GeneID:3934 |
| GDPD1 | GeneID:284161 | | VLDLR | GeneID:7436 |
| NMU | GeneID:10874 | | DIAPH2 | GeneID:1730 |
| MARCH8 | GeneID:220972 | | HUNK | GeneID:30811 |
| KLK10 | GeneID:5655 | | RNF144A | GeneID:9781 |
| TFF3 | GeneID:7033 | | FAM84A | GeneID:151354 |
| CREB3L1 | GeneID:90993 | | PKP1 | GeneID:5317 |
| C14orf132 | GeneID:56967 | | SDC2 | GeneID:6383 |
| CXADR | GeneID:1525 | | NPR3 | GeneID:4883 |
| NUPR1 | GeneID:26471 | | CSRP2 | GeneID:1466 |
| WNT4 | GeneID:54361 | | KAL1 | GeneID:3730 |
| UGT1A4 | GeneID:54657 | | RAI14 | GeneID:26064 |
| GRHL3 | GeneID:57822 | | ACPL2 | GeneID:92370 |
| GALNTL4 | GeneID:374378 | | DHRS3 | GeneID:9249 |
| PDE4D | GeneID:5144 | | PRIMA1 | GeneID: 145270 |
| CXCR4 | GeneID:7852 | | DKK1 | GeneID:22943 |
| ITGA6 | GeneID:3655 | | BCAS1 | GeneID:8537 |
| CRYBG3 | GeneID:131544 | | VIM | GeneID:7431 |
| SYTL4 | GeneID:94121 | | KLF13 | GeneID:51621 |
| PCDH9 | GeneID:5101 | | C21orf63 | GeneID:59271 |
| CTSC | GeneID:1075 | | GFRA1 | GeneID:2674 |
| MNS1 | GeneID:55329 | | SLC16A1 | GeneID:6566 |
| CYFIP2 | GeneID:26999 | | COL12A1 | GeneID:1303 |
| LXN | GeneID:56925 | | FAM26F | GeneID:441168 |
| RPS6KA3 | GeneID:6197 | | KYNU | GeneID:8942 |
| KCNMA1 | GeneID:3778 | | PDP2 | GeneID:57546 |
| RCN1 | GeneID:5954 | | SYNGR1 | GeneID:9145 |
| LYPD1 | GeneID:116372 | | RHOBTB3 | GeneID:22836 |
| PCP4 | GeneID:5121 | | STC1 | GeneID:6781 |
| PLA2G4C | GeneID:8605 | | EPN2 | GeneID:22905 |
| HS6ST2 | GeneID:90161 | | NFKBIZ | GeneID:64332 |
| TIAM1 | GeneID:7074 | | PTGES | GeneID:9536 |
| AMOTL1 | GeneID:154810 | | KCNE4 | GeneID:23704 |
| ACOX2 | GeneID:8309 | | TFPI | GeneID:7035 |
| EPB41L4A | GeneID:64097 | | NT5C3L | GeneID:115024 |
| C11orf75 | GeneID:56935 | | PMP22 | GeneID:5376 |
| TMEM45B | GeneID: 120224 | | APOBEC3B | GeneID:9582 |
| HIPK2 | GeneID:28996 | | C8orf57 | GeneID:84257 |
| HSD1788 | GeneID:7923 | | HSPB8 | GeneID:26353 |
| PARP9 | GeneID:83666 | | C10orf112 | GeneID:340895 |
| DDIT4 | GeneID:54541 | | ADAMTS19 | GeneID:171019 |
| TNS3 | GeneID:64759 | | COL4A5 | GeneID:1287 |
| MORC4 | GeneID:79710 | | PUNC | GeneID:9543 |
| CDK6 | GeneID:1021 | | MMP16 | GeneID:4325 |
| TMPRSS4 | GeneID:56649 | | UGT8 | GeneID:7368 |
| BACE2 | GeneID:25825 | | NECAB1 | GeneID:64168 |
| EPAS1 | GeneID:2034 | | KCNK2 | GeneID:3776 |
| FJX1 | GeneID:24147 | | SYNPO2 | GeneID: 171024 |
| PRR15 | GeneID:222171 | | WIPF1 | GeneID:7456 |
| SRI | GeneID:6717 | | | |
| TGFB3 | GeneID:7043 | | | |
wherein the induction and/or downregulation of said genes are indicative of the expression of the ZNF703 gene.

15. The method of claim 1, wherein said over-expression of the ZNF703 gene occurs when the transcription and/or the translation of the gene leads to an expression level in a biological sample that is at least 20% superior to the normal level of expression of said gene.

16. The method of claim 1, wherein said over-expression of the ZNF703 gene occurs when the transcription and/or the translation of the gene leads to an expression level in a biological sample that is at least 50% superior to the normal level of expression of said gene.

17. The method of claim 1, wherein said over-expression of the ZNF703 gene occurs when the transcription and/or the translation of the gene leads to an expression level in a biological sample that is at least 100% superior to the normal level of expression of said gene.

18. The method of claim 1, wherein said method further comprises the step of comparing the level of expression of the ZNF703 gene in a biological sample from a female with its expression level in a control.

19. The method of claim 18 wherein said control is a control sample comprising non-tumoral cells.

20. The method of claim 18 wherein said control is a control sample comprising normal breast tissues.

21. Use of a kit comprising at least one nucleic acid probe or oligonucleotide or at least one antibody for determining the copy number of the ZNF703 gene, and/or determining the expression of the ZNF703 gene for diagnosing a breast cancer of the luminal-B subtype in a female.

## Patentansprüche

1. Eine In-Vitro-Methode zur Diagnose von Brustkrebs des Luminal-B Subtyps bei einer Frau. Die Methode umfasst mehrere Schritte für die Analyse von biologischen Proben von besagter Frau:
i) Feststellen der Vervielfältigungszahl des Zink-Finger-Protein-703-Gens (ZNF703), und/oder
ii) Feststellung der Expression des ZNF703-Gens
wobei eine erhöhte Vervielfältigungszahl und/oder eine Überexpression des ZNF703-Gens zeigt einen Luminal-B Tumor an.

2. Die Methode des Antrags 1, wobei besagter Luminal-B Tumor mit einer schlechten Diagnose in Verbindung steht.

3. Die Methode von Antrag 1, wobei eine erhöhte Vervielfältigungszahl und/oder eine Überexpression des ZNF703-Gens eine Erhöhung der Population der Krebs-Stammzellen (CSC) anzeigt.

4. Die Methode von Antrag 1, wobei Anti-Östrogene nicht als Behandlung für die mit Brustkrebs des Luminal-B Subtyps diagnostizierte Frau ausgewählt werden, sondern stattdessen aggressivere Behandlungen wie Chemotherapie für die mit Brustkrebs des Luminal-B Subtyps diagnostizierte Frau ausgewählt werden.

5. Die Methode von Antrag 1, wobei es sich um eine Frau handelt.

6. Die Methode von Antrag 1, wobei die besagte Frau vermutlich Brustkrebs bekommt oder bereits Brustkrebs entwickelt.

7. Die Methode von Antrag 1, wobei die biologischen Proben eine Brustgewebeprobe ist.

8. Die Methode von Antrag 1, wobei die biologische Probe eine Brusttumorprobe ist.

9. Die Methode von Antrag 1, wobei besagte Methode die Bestimmung der Vervielfältigungszahl der ZNF703-Gene umfasst.

10. Die Methode von Antrag 1, wobei besagte Vervielfältigungszahl des ZNF703-Gens sich auf die Vervielfältigungszahl des ZNF703-Gens durch Genome bezieht, welches stärker ist als die zwei Allele des ZNF703-Gens.

11. Die Methode von Antrag 1, wobei besagte Methode die Feststellung der Expression des ZNF703-Gens umfasst.

12. Die Methode von Antrag 11, wobei besagter Analyseschritt durch die Analyse der Expression des mRNA-Transkripts oder mRNA-Präkusors von besagtem Gen, bewertet wird.

13. Die Methode von Antrag 11, wobei besagter Analyseschritt durch die Analyse der Expression des ZNF703-Proteins, das aus besagtem Gen übertragen wurde, bewertet wird.

14. Eine In-Vitro-Methode für die Diagnose von Brustkrebs des Luminal-B-Subtyps bei einer Frau. Die Methode umfasst den Analyseschritt einer biologischen Probe von besagter Frau durch die Bestimmung der Expression des ZNF703-Gens, wobei eine Überexpression des ZNF703-Gens einen Luminal B-Tumor anzeigt. Hierbei wird besagter Analyseschritt indirekt über die Bestimmung des Expression von wenigstens 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185 oder189 Genen der 189 Gene, die in Tabelle 1 aufgeführt sind, bewertet:
| Gen-Symbol | Gen-ID.MAJ | | MID1 | Gen-ID:4281 |
|---|---|---|---|---|
| IGFBP5 | Gen-ID:3488 | | LAMP3 | Gen-ID:27074 |
| ASCL1 | Gen-ID:429 | | DOCK11 | Gen-ID:139818 |
| TOX3 | Gen-ID:27324 | | PLS3 | Gen-ID:5358 |
| CLCA2 | Gen-ID:9635 | | HEY2 | Gen-ID:23493 |
| FU25076 | Gen-ID:134111 | | SLC1A1 | Gen-ID:6505 |
| GOLM1 | Gen-ID:51280 | | EHF | Gen-ID:26298 |
| LAMB1 | Gen-ID:3912 | | PDE4B | Gen-ID:5142 |
| SCIN | Gen-ID:85477 | | CYP4Z1 | Gen-ID:199974 |
| PKIA | Gen-ID:5569 | | LRP12 | Gen-ID:29967 |
| LDHB | Gen-ID:3945 | | TLE4 | Gen-ID:7091 |
| KCNJ8 | Gen-ID:3764 | | FAM129A | Gen-ID:116496 |
| SLITRK6 | Gen-ID:84189 | | CAV1 | Gen-ID:857 |
| SOCS2 | Gen-ID:8835 | | WDR72 | Gen-ID:256764 |
| PRSS23 | Gen-ID:11098 | | LEF1 | Gen-ID:51176 |
|---|---|---|---|---|
| CYBRD1 | Gen-ID:79901 | | IGFBP3 | Gen-ID:3486 |
| ADD3 | Gen-ID:120 | | MARCKS | Gen-ID:4082 |
| ELF5 | Gen-ID:2001 | | CHN2 | Gen-ID:1124 |
| TRGC2 | Gen-ID:6967 | | B3GNT5 | Gen-ID:84002 |
| LYN | Gen-ID:4067 | | RAP1A | Gen-ID:5906 |
| FHL1 | Gen-ID:2273 | | STEAP4 | Gen-ID:79689 |
| CAV2 | Gen-ID:858 | | BMP7 | Gen-ID:655 |
| FABP5 | Gen-ID:2171 | | BDKRB2 | Gen-ID:624 |
| SASH1 | Gen-ID:23328 | | FN1 | Gen-ID:2335 |
| GALNT12 | Gen-ID:79695 | | PTGER4 | Gen-ID:5734 |
| TCF12 | Gen-ID:6938 | | DSC2 | Gen-ID:1824 |
| AOX1 | Gen-ID:316 | | NTN1 | Gen-ID:9423 |
| UGT1A10 | Gen-ID:54575 | | SUSD4 | Gen-ID:55061 |
| KCTD12 | Gen-ID:115207 | | PPAP2A | Gen-ID:8611 |
| GP2 | Gen-ID:2813 | | KLK5 | Gen-ID:25818 |
| PKIB | Gen-ID:5570 | | SUSD2 | Gen-ID:56241 |
| SOX2 | Gen-ID:6657 | | HERC5 | Gen-ID:51191 |
| LMO3 | Gen-ID:55885 | | RCAN1 | Gen-ID:1827 |
| MUCLI | Gen-ID:118430 | | MET | Gen-ID:4233 |
| MUMIL1 | Gen-ID:139221 | | BTBD11 | Gen-ID:121551 |
| TMSL8 | Gen-ID:11013 | | MMD | Gen-ID:23531 |
| FGF13 | Gen-ID:2258 | | PRICKLE1 | Gen-ID:144165 |
| SGCE | Gen-ID:8910 | | UBE2L6 | Gen-ID:9246 |
| GOLSYN | Gen-ID:55638 | | SLC27A2 | Gen-ID:11001 |
| CALML5 | Gen-ID:51806 | | XK | Gen-ID:7504 |
| JAZF1 | Gen-ID:221895 | | MOBKL2B | Gen-ID:79817 |
| ANKH | Gen-ID:56172 | | GRB14 | Gen-ID:2888 |
| ATP8A2 | Gen-ID:51761 | | FAR2 | Gen-ID:55711 |
| ALDH1A3 | Gen-ID:220 | | TES | Gen-ID:26136 |
| BHLHB3 | Gen-ID:79365 | | PMEPA1 | Gen-ID:56937 |
|---|---|---|---|---|
| RIN2 | Gen-ID:54453 | | LCN2 | Gen-ID:3934 |
| GDPD1 | Gen-ID:284161 | | VLDLR | Gen-ID:7436 |
| NMU | Gen-ID:10874 | | DIAPH2 | Gen-ID:1730 |
| MARCH8 | Gen-ID:220972 | | HUNK | Gen-ID:30811 |
| KLK10 | Gen-ID:5655 | | RNF144A | Gen-ID:9781 |
| TFF3 | Gen-ID:7033 | | FAM84A | Gen-ID:151354 |
| CREB3L1 | Gen-ID:90993 | | PKP1 | Gen-ID:5317 |
| C14orf132 | Gen-ID:56967 | | SDC2 | Gen-ID:6383 |
| CXADR | Gen-ID:1525 | | NPR3 | Gen-ID:4883 |
| NUPR1 | Gen-ID:26471 | | CSRP2 | Gen-I D:1466 |
| WNT4 | Gen-ID:54361 | | KAL1 | Gen-ID:3730 |
| UGT1A4 | Gen-ID:54657 | | RAI14 | Gen-ID:26064 |
| GRHL3 | Gen-ID:57822 | | ACPL2 | Gen-ID:92370 |
| GALNTL4 | Gen-ID:374378 | | DHRS3 | Gen-ID:9249 |
| PDE4D | Gen-ID:5144 | | PRIMA1 | Gen-ID:145270 |
| CXCR4 | Gen-ID:7852 | | DKK1 | Gen-ID:22943 |
| ITGA6 | Gen-ID:3655 | | BCAS1 | Gen-ID:8537 |
| CRYBG3 | Gen-ID:131544 | | VIM | Gen-ID:7431 |
| SYTL4 | Gen-ID:94121 | | KLF13 | Gen-ID:51621 |
| PCDH9 | Gen-ID:5101 | | C21orf63 | Gen-ID:59271 |
| CTSC | Gen-ID:1075 | | GFRA1 | Gen-ID:2674 |
| MNS1 | Gen-ID:55329 | | SLC16A1 | Gen-ID:6566 |
| CYFIP2 | Gen-ID:26999 | | COL12A1 | Gen-ID:1303 |
| LXN | Gen-ID:56925 | | FAM26F | Gen-ID:441168 |
| RPS6KA3 | Gen-ID:6197 | | KYNU | Gen-ID:8942 |
| KCNMA1 | Gen-ID:3778 | | PDP2 | Gen-ID:57546 |
| RCN1 | Gen-ID:5954 | | SYNGR1 | Gen-ID:9145 |
| LYPD1 | Gen-ID:116372 | | RHOBTB3 | Gen-ID:22836 |
| PCP4 | Gen-ID:5121 | | STC1 | Gen-ID:6781 |
| PLA2G4C | Gen-ID:8605 | | EPN2 | Gen-ID:22905 |
| HS6ST2 | Gen-ID:90161 | | NFKBIZ | Gen-ID:64332 |
|---|---|---|---|---|
| TIAM1 | Gen-ID:7074 | | PTG ES | Gen-ID:9536 |
| AMOTL1 | Gen-ID:154810 | | KCNE4 | Gen-ID:23704 |
| ACOX2 | Gen-ID:8309 | | TFPI | Gen-ID:7035 |
| EPB41L4A | Gen-ID:64097 | | NT5C3L | Gen-ID:115024 |
| C11orf75 | Gen-ID:56935 | | PMP22 | Gen-ID:5376 |
| TMEM45B | Gen-ID:120224 | | APOBEC3B | Gen-ID:9582 |
| HIPK2 | Gen-ID:28996 | | C8orf57 | Gen-ID:84257 |
| HSD17B8 | Gen-ID:7923 | | HSPB8 | Gen-ID:26353 |
| PARP9 | Gen-ID:83666 | | C10orf112 | Gen-ID:340895 |
| DDIT4 | Gen-ID:54541 | | ADAMTS19 | Gen-ID:171019 |
| TNS3 | Gen-ID:64759 | | COL4A5 | Gen-ID:1287 |
| MORC4 | Gen-ID:79710 | | PUNC | Gen-ID:9543 |
| CDK6 | Gen-ID:1021 | | MMP16 | Gen-ID:4325 |
| TMPRSS4 | Gen-ID:56649 | | UGT8 | Gen-ID:7368 |
| BACE2 | Gen-ID:25825 | | NECAB1 | Gen-ID:64168 |
| EPAS1 | Gen-ID:2034 | | KCNK2 | Gen-ID:3776 |
| FJX1 | Gen-ID:24147 | | SYNP02 | Gen-ID:171024 |
| PRR15 | Gen-ID:222171 | | WIPF1 | Gen-ID:7456 |
| SRI | Gen-ID:6717 | | | |
| TGFB3 | Gen-ID:7043 | | | |
wobei die Induktion und/oder das Herunterregulieren von besagten Genen ein Anzeichen für die Expression des ZNF703-Gens sind.

15. Die Methode von Antrag 1, wobei besagte Überexpression des ZNF703-Gens auftritt, wenn die Transkription und/oder die Übertragung des Gens zu einem Expressionsniveau in der biologischen Probe führen, das wenigstens 20% stärker ist als das normale Expressionsniveau von besagtem Gen.

16. Die Methode von Antrag 1, wobei besagte Überexpression des ZNF703-Gens geschieht, wenn die Transkription und/oder die Übertragung des Gens zu einem Expressionsniveau in der biologischen Probe führen, das wenigstens 50% stärker ist als das normale Expressionsniveau von besagtem Gen.

17. Die Methode von Antrag 1, wobei besagte Überexpression des ZNF703-Gens geschieht, wenn die Transkription und/oder die Übertragung des Gens zu einem Expressionsniveau in der biologischen Probe führen, das wenigstens 100% stärker ist als das normale Expressionsniveau von besagtem Gen.

18. Die Methode von Antrag 1, wobei besagte Methode des Weiteren den Arbeitsschritt des Vergleichs des Expressionsniveaus des ZNF703-Gens in einer biologischen Probe von einer Frau umfasst, und es sich um das Expressionsniveau in einer Kontrollprobe handelt.

19. Die Methode von Antrag 18, wobei besagte Kontrolle, eine Kontrollprobe ohne Krebszellen umfasst.

20. Die Methode von Antrag 18, wobei die Kontrolle eine Kontrollprobe umfasst, die aus normalem Brustgewebe besteht.

21. Die Verwendung eines Sets, das wenigstens eine Nukleinsäuren/Oligonukleotid -Sonde oder wenigstens einen Antikörper für die Bestimmung der Vervielfältigungszahl des ZNF703-Gens und/oder der Bestimmung der Expression des ZNF703-Gens für die Diagnose von Brustkrebs des Luminal-B Subtyps bei einer Frau umfasst.

## Revendications

1. Un procédé in vitro pour le diagnostic d'un cancer du sein du sous-type luminal B chez une femelle, comprenant les étapes de l'analyse d'un échantillon biologique de la dite femelle consistant à :
i) déterminer le nombre de copies du gène à protéine à doigt de zinc 703 (gène ZNF703), et / ou
ii) à déterminer l'expression du gène ZNF703
dans lequel une augmentation du nombre de copies et / ou une surexpression du gène ZNF703 sont indicatives d'une tumeur luminale B.

2. Le procédé de la revendication 1, dans lequel la dite tumeur luminale B est associée à un pronostic défavorable.

3. Le procédé de la revendication 1, dans lequel une augmentation du nombre de copies et / ou une surexpression du gène ZNF703 sont indicatives d'une augmentation de la population de Cellules Souches du Cancer (CSC)

4. Le procédé de la revendication 1, dans lequel des anti-oestrogènes ne sont pas sélectionnés comme traitement chez la femme faisant l'objet d'un diagnostic de cancer du sein du sous-type luminal B, mais des traitements plus agressifs, tels que la chimiothérapie, sont sélectionnés pour la femme faisant l'objet d'un diagnostic de cancer du sein du sous-type luminal B

5. Le procédé de la revendication 1, dans lequel la femelle est une femme.

6. Le procédé de la revendication 1, dans lequel il est estimé que la dite femelle est sur le point de développer ou développe un cancer du sein.

7. Le procédé de la revendication 1, dans lequel l'échantillon biologique est un échantillon de tissu du sein.

8. Le procédé de la revendication 1, dans lequel l'échantillon biologique est un échantillon de tumeur du sein.

9. Le procédé de la revendication 1, dans lequel le dit procédé comprend l'étape consistant à déterminer le nombre de copies du gène ZNF703.

10. Le procédé de la revendication 1, dans lequel la dite augmentation du nombre de copies du gène ZNF703 se rapporte à un nombre de copies du gène ZNF703 par génome, supérieur aux deux allèles du gène ZNF703.

11. Le procédé de la revendication 1, dans lequel le dit procédé comprend une étape consistant à déterminer l'expression du gène ZNF703.

12. Le procédé de la revendication 11, dans lequel la dite étape de détermination est évaluée en analysant l'expression d'un transcrit ARNm ou de précurseurs ARNm du dit gène.

13. Le procédé de la revendication 11, dans lequel la dite étape de détermination est évaluée en analysant l'expression de la protéine ZNF703 traduite à partir de ce gène.

14. Un procédé in vitro pour le diagnostic d'un cancer du sein du sous-type luminal B chez une femelle, comprenant les étapes consistant à analyser un échantillon biologique prélevé sur la dite femelle en déterminant l'expression du gène ZNF703, dans lequel une surexpression du gène ZNF703 est indicative d'une tumeur luminale B et dans lequel la dite étape de détermination est évaluée indirectement en déterminant l'expression d'au moins 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185 ou 189 des 189 gènes listés dans le tableau 1 :
| Symbole gène | N° id. Gene MAJ | | MID1 | N° id. gène : 4281 |
|---|---|---|---|---|
| IGFBP5 | N° id. gène : 3488 | | LAMP3 | N° id. gène : 27074 |
| ASCL1 | N° id. gène : 429 | | DOCK 11 | N° id. gène : 139818 |
| TOX3 | N° id. gène : 27324 | | PLS3 | N° id. gène : 5358 |
| CLCA2 | N° id. gène : 9635 | | HEY2 | N° id. gène : 23493 |
| FLJ25076 | N° id. gène : 134111 | | SLC1AI | N° id. gène : 6505 |
| GOLM1 | N° id. gène : 51280 | | EHF | N° id. gène : 26298 |
| LAMB1 | N° id. gène : 3912 | | PDE4B | N° id. gène : 5142 |
| SCIN | N° id. gène : 85477 | | CYP4Z1 | N° id. gène : 199974 |
| PKIA | N° id. gène : 5569 | | LRP12 | N° id. gène : 29967 |
| LDHB | N° id. gène : 3945 | | TLE4 | N° id. gène : 7091 |
| KCNJ8 | N° id. gène : 3764 | | FAM129A | N° id. gène : 116496 |
| SLITRK6 | N° id. gène : 84189 | | CAV1 | N° id. gène : 857 |
| SOCS2 | N° id. gène : 8835 | | WDR72 | N° id. gène : 256764 |
| PRSS23 | N° id. gène : 11098 | | LEF1 | N° id. gène : 51176 |
| CYBRD1 | N° id. gène : 79901 | | IGFBP3 | N° id. gène : 3486 |
| ADD3 | N° id. gène : 120 | | MARCKS | N° id. gène : 4082 |
| ELF5 | N° id. gène : 2001 | | CHN2 | N° id. gène : 1124 |
| TRGC2 | N° id. gène : 6967 | | B3GNT5 | N° id. gène : 84002 |
| LYN | N° id. gène : 4067 | | RAP1A | N° id. gène : 5906 |
| FHL1 | N° id. gène : 2273 | | STEAP4 | N° id. gène : 79689 |
| CAV2 | N° id. gène : 858 | | BMP7 | N° id. gène : 655 |
| FABP5 | N° id. gène : 2171 | | BDKRB2 | N° id. gène : 624 |
| SASH1 | N° id. gène : 23328 | | FN1 | N° id. gène : 2335 |
| GALNT12 | N° id. gène : 79695 | | PTGER4 | N° id. gène : 5734 |
| TCF12 | N° id. gène : 6938 | | DSC2 | N° id. gène : 1824 |
| AOX1 | N° id. gène : 316 | | NTN1 | N° id. gène : 9423 |
| UGT1A10 | N° id. gène : 54575 | | SUSD4 | N° id. gène : 55061 |
| KCTD12 | N° id. gène :115207 | | PPAP2A | N° id. gène : 8611 |
| GP2 | N° id. gène : 2813 | | KLK5 | N° id. gène : 25818 |
| PKIB | N° id. gène : 5570 | | SUSD2 | N° id. gène : 56241 |
| SOX2 | N° id. gène : 6657 | | HERC5 | N° id. gène : 51191 |
| LM03 | N° id. gène : 55885 | | RCAN1 | N° id. gène : 1827 |
| MUCLI | N° id. gène :118430 | | MET | N° id. gène : 4233 |
| MUMIL1 | N° id. gène : 139221 | | BTBD11 | N° id. gène : 121551 |
| TMSL8 | N° id. gène : 11013 | | MMD | N° id. gène : 23531 |
| FGF13 | N° id. gène : 2258 | | PRICKLE1 | N° id. gène : 144165 |
| SGCE | N° id. gène : 8910 | | UBE2L6 | N° id. gène : 9246 |
| GOLSYN | N° id. gène: 55638 | | SLC27A2 | N° id. gène : 11001 |
| CALML5 | N° id. gène : 51806 | | XK | N° id. gène : 7504 |
| JAZF1 | N° id. gène : 221895 | | MOBKL2B | N° id. gène : 79817 |
| ANKH | N° id. gène : 56172 | | GRB14 | N° id. gène : 2888 |
| ATP8A2 | N° id. gène : 51761 | | FAR2 | N° id. gène : 55711 |
| ALDH1A3 | N° id. gène : 220 | | TES | N° id. gène : 26136 |
| BHLHB3 | N° id. gène : 79365 | | PMEPA1 | N° id. gène : 56937 |
| RIN2 | N° id. gène : 54453 | | LCN2 | N° id. gène : 3934 |
| GDPD1 | N° id. gène: 284161 | | VLDLR | N° id. gène : 7436 |
| NMU | N° id. gène : 10874 | | D1APH2 | N° id. gène : 1730 |
| MARCH8 | N° id. gène : 220972 | | HUNK | N° id. gène : 30811 |
| KLK10 | N° id. gène : 5655 | | RNF144A | N° id. gène : 9781 |
| TFF3 | N° id. gène : 7033 | | FAM84A | N° id. gène : 151354 |
| CREB3L1 | N° id. gène : 90993 | | PKP1 | N° id. gène : 5317 |
| C14orf132 | N° id. gène : 56967 | | SDC2 | N° id. gène : 6383 |
| CXADR | N° id. gène : 1525 | | NPR3 | N° id. gène : 4883 |
| NUPR1 | N° id. gène : 26471 | | CSRP2 | N° id. gène : 1466 |
| WNT4 | N° id. gène : 54361 | | KALI | N° id. gène : 3730 |
| UGT1A4 | N° id. gène : 54657 | | RAI14 | N° id. gène : 26064 |
| GRHL3 | N° id. gène : 57822 | | ACPL2 | N° id. gène : 92370 |
| GALNTL4 | N° id. gène : 374378 | | DHRS3 | N° id. gène : 9249 |
| PDE4D | N° id. gène : 5144 | | PRIMA1 | N° id. gène : 145270 |
| CXCR4 | N° id. gène : 7852 | | DKK1 | N° id. gène : 22943 |
| ITGA6 | N° id. gène : 3655 | | BCAS1 | N° id. gène : 8537 |
| CRYBG3 | N° id. gène : 131544 | | VIM | N° id. gène : 7431 |
| SYTL4 | N° id. gène : 94121 | | KLF13 | N° id. gène : 51621 |
| PCDH9 | N° id. gène : 5101 | | C21orf63 | N° id. gène : 59271 |
| CTSC | N° id. gène : 1075 | | GFRA1 | N° id. gène : 2674 |
| MNS1 | N° id. gène : 55329 | | SLC16A1 | N° id. gène : 6566 |
| CYFIP2 | N° id. gène : 26999 | | COL12AI | N° id. gène : 1303 |
| LXN | N° id. gène : 56925 | | FAM26F | N° id. gène : 441168 |
| RPS6KA3 | N° id. gène : 6197 | | KYNU | N° id. gène : 8942 |
| KCNMA1 | N° id. gène : 3778 | | PDP2 | N° id. gène : 57546 |
| RCN1 | N° id. gène : 5954 | | SYNGR1 | N° id. gène : 9145 |
| LYPD1 | N° id. gène : 116372 | | RHOBTB3 | N° id. gène : 22836 |
| PCP4 | N° id. gène : 5121 | | STC1 | N° id. gène : 6781 |
| PLA2G4C | N° id. gène : 8605 | | EPN2 | N° id. gène : 22905 |
| HS6ST2 | N° id. gène : 90161 | | NFKBIZ | N° id. gène : 64332 |
| TIAM1 | N° id. gène : 7074 | | PTGES | N° id. gène : 9536 |
| AMOTL1 | N° id. gène : 154810 | | KCNE4 | N° id. gène : 23704 |
| ACOX2 | N° id. gène : 8309 | | TFPI | N° id. gène : 7035 |
| EPB41L4A | N° id. gène : 64097 | | NT5C3L | N° id. gène : 115024 |
| C11orf75 | N° id. gène : 56935 | | PMP22 | N° id. gène : 5376 |
| TMEM45B | N° id. gène : 120224 | | APOBEC3B | N° id. gène : 9582 |
| HIPK2 | N° id. gène: 28996 | | C8orf57 | N° id. gène: 84257 |
|---|---|---|---|---|
| HSD17B8 | N° id. gène: 7923 | | HSPB8 | N° id. gène : 26353 |
| PARP9 | N° id. gène : 83666 | | C10orf112 | N° id. gène : 340895 |
| DDIT4 | N° id. gène : 54541 | | ADAMTS19 | N° id. gène : 171019 |
| TNS3 | N° id. gène : 64759 | | COL4A5 | N° id. gène : 1287 |
| MORC4 | N° id. gène: 79710 | | PUNC | N° id. gène : 9543 |
| CDK6 | N° id. gène: 1021 | | MMP16 | N° id. gène : 4325 |
| TMPRSS4 | N° id. gène : 56649 | | UGT8 | N° id. gène : 7368 |
| BACE2 | N° id. gène : 25825 | | NECAB1 | N° id. gène : 64168 |
| EPAS1 | N° id. gène : 2034 | | KCNK2 | N° id. gène : 3776 |
| FJX1 | N° id. gène : 24147 | | SYNP02 | N° id. gène : 171024 |
| PRR15 | N° id. gène : 222171 | | WIPFI | N° id. gène : 7456 |
| SRI | N° id. gène : 6717 | | | |
| TGFB3 | N° id. gène : 7043 | | | |
dans lequel l'induction et / ou la régulation à la baisse des dits gènes sont indicatives de l'expression du gène ZNF703.

15. Le procédé de la revendication 1, dans lequel la dite surexpression du gène ZNF703 a lieu quand la transcription et / ou la traduction du gène aboutit à un niveau d'expression dans un échantillon biologique supérieur d'au moins 20 % au niveau d'expression normal du dit gène.

16. Le procédé de la revendication 1, dans lequel la dite surexpression du gène ZNF703 a lieu quand la transcription et / ou la traduction du gène aboutit à un niveau d'expression dans un échantillon biologique supérieur d'au moins 50 % au niveau d'expression normal du dit gène.

17. Le procédé de la revendication 1, dans lequel la dite surexpression du gène ZNF703 a lieu quand la transcription et / ou la traduction du gène aboutit à un niveau d'expression dans un échantillon biologique supérieur d'au moins 100 % au niveau d'expression normal du dit gène.

18. Le procédé de la revendication 1, dans lequel le dit procédé comprend par ailleurs une étape consistant à comparer le niveau d'expression du gène ZNF703 dans un échantillon biologique prélevé sur une femelle avec son niveau d'expression dans un contrôle.

19. Le procédé de la revendication 18 dans lequel le dit contrôle est un échantillon de contrôle comprenant des cellules non tumorales.

20. Le procédé de la revendication 18 dans lequel le dit contrôle est un échantillon de contrôle comprenant des tissus du sein normaux.

21. Utilisation d'un kit comprenant au moins une sonde d'acide nucléique ou oligonucléotide ou au moins un anticorps pour déterminer le nombre de copies du gène ZNF703 et / ou déterminer l'expression du gène ZNF703 pour le diagnostic d'un cancer du sein du sous-type luminal B chez une femelle.
